# EUROPÄISCHE PATENTSCHRIFT

(11) **EP 0 548 826 B1**
(45) Veröffentlichungstag und Bekanntmachung des Hinweises auf die Patenterteilung: **23.04.1997**
(21) Anmeldenummer: 92121560.4
(22) Anmeldetag: 18.12.1992
(51) Int. Cl.: C07C 211/21, C07F 5/02, G03G 9/097

(54) **Diallylammonium-Verbindungen, Verfahren zu ihrer Herstellung sowie ihre Verwendung**
Diallyl ammonium-compounds, method for their preparation and their application
Composés de diallylammonium, leur procédé de préparation et leur utilisation

(30) Priorität: 21.12.1991 DE 4142541
(43) Veröffentlichungstag der Anmeldung: 30.06.1993
(73) Patentinhaber: HOECHST AKTIENGESELLSCHAFT, 65926 Frankfurt am Main (DE)
(72) Erfinder: Baur, Rüdiger, Dr., W-6239 Eppstein/Ts. (DE); Macholdt, Hans-Tobias, Dr., W-6100 Darmstadt-Eberstadt (DE)

(56) Entgegenhaltungen:
- EP-A- 0 053 888
- EP-A- 0 284 000
- DD-A- 225 128
- US-A- 4 400 396
- DATABASE WPI, Week 8512, Derwent Publications Ltd., London, GB; AN 85071801; & JP-A-26 346
- DATABASE WPI, Week 8601, Derwent Publications Ltd., London, GB; AN 86-004301; JP-A-60 231 595
- J. CHROMATOGR., 399, 47-67, 1987, K. G. FURTON et al: "Thermodynamic characteristics of solute-solvent interactions in liquid organic salt solvents, studied by gas chromatography"
- POLYHEDRON, 4(7), 1269-70, 1985, C. DIAZ: "A characteristic redox reaction for N,N'-thiobisamines and some other S(II) compounds"
- BULLETIN DE LA SOCIETE CHIMIQUE DE FRANCE, 1962, Paris, FR, Seite 933-937, J. VAN OVERBEKE et al: "Halogénures de tétrahydropyrannylammonium quaternaires"
- JOURNAL OF PHYSICAL CHEMISTRY, Bd. 66, 1962, Easton, US, Seite 2149-2154, TH. E. MEAD: "Heats of Neutralization and relative strengths of amines in benzene"
- CHEMICAL ABSTRACTS, vol. 61, no. 10, 9. November 1964, Columbus, Ohio, US; S.P. MISKIDZH'YAN et al: "Conducting binary, nonaqueous systems in a third component", Spalte 11386f
- CHEMICAL ABSTRACTS, vol. 89 Columbus, Ohio, US; abstract no. 107937a, A. A. STOTSKII et al: "Preparation and nitration of dialkylamine nitrates"
- PATENT ABSTRACTS OF JAPAN, vol. 8, no. 188 (P-297)(1625), 29. August 1984; & JP-A-59 78 364
- PATENT ABSTRACTS OF JAPAN, vol. 12, no. 231, (P-723)(3078), 30. Juni 1988; & JP-A-63 23 167
- PATENT ABSTRACTS OF JAPAN, vol. 10, no. 348 (P-519)(2404), 22. November 1986; & JP-A-61 147 260
- PATENT ABSTRACTS OF JAPAN, vol. 11, no. 109 (P-564)(2556), 7. April 1987; & JP-A-61 258 270
- PATENT ABSTRACTS OF JAPAN, vol. 13, no. 27 (P-815)(3375), 20. Januar 1989; & JP-A-63 226 665

## Beschreibung

Die vorliegende Erfindung liegt auf dem technischen Gebiet der Ladungssteuermittel in Tonern und Entwicklern für elektrophotographische Aufzeichnungsverfahren sowie in Pulvern und Pulverlacken zur Oberflächenbeschichtung.

Bei elektrophotographischen Aufzeichnungsverfahren wird auf einem Photoleiter ein "latentes Ladungsbild" erzeugt. Dies erfolgt beispielsweise durch Aufladung eines Photoleiters durch eine Coronaentladung und anschließende bildmäßige Belichtung der elektrostatisch aufgeladenen Oberfläche des Photoleiters, wobei durch die Belichtung der Ladungsabfluß zur geerdeten Unterlage an den belichteten Stellen bewirkt wird.
Anschließend wird das so erzeugte "latente Ladungsbild" durch Aufbringen eines Toners entwickelt. Im nächsten Schritt wird der Toner vom Photoleiter auf beispielsweise Papier, Textilien, Folien oder Kunststoff übertragen und beispielsweise mittels Druck, Strahlung, Hitze oder Lösungsmitteleinwirkung fixiert. Der benutzte Photoleiter wird anschließend gereinigt und steht für einen neuen Aufzeichnungsvorgang zur Verfügung.

In zahlreichen Patentschriften wird die Optimierung von Tonern beschrieben, wobei u. a. der Einfluß des Tonerbindemittels (Variation von Harz/Harzkomponenten oder Wachs/Wachskomponenten), der Einfluß von Carriern (bei Zweikomponentenentwicklern) und Magnetpigmenten (bei Einkomponentenentwicklern) untersucht werden.

Ein Maß für die Tonerqualität ist seine spezifische Aufladung q/m (Ladung pro Masseeinheit). Neben Vorzeichen und Höhe der elektrostatischen Aufladung ist vor allem das schnelle Erreichen der gewünschten Ladungshöhe und die Konstanz dieser Ladung über einen längeren Aktivierzeitraum hinweg ein entscheidendes Oualitätskriterium. In der Praxis ist dies insofern von zentraler Bedeutung, als daß der Toner im Entwicklergemisch, bevor er auf den Photoleiter übertragen wird, einer erheblichen Aktivierzeit ausgesetzt sein kann, da er teilweise über einen Zeitraum der Herstellung von bis zu mehreren tausend Kopien im Entwicklergemisch verbleibt. Darüberhinaus ist die Unempfindlichkeit des Toners gegen Klimaeinflüsse, wie Temperatur und Luftfeuchtigkeit, ein weiteres wichtiges Eignungskriterium.

Sowohl positiv als auch negativ aufladbare Toner finden Verwendung in Kopierern und Laserdruckern in Abhängigkeit vom Verfahrens- und Gerätetyp.

Um elektrophotographische Toner oder Entwickler mit entweder positiver oder negativer Aufladung zu erhalten, werden häufig sogenannte Ladungssteuermittel (auch Ladungskontrollmittel genannt) zugesetzt. Dabei ist neben dem Vorzeichen der Ladungssteuerung das Ausmaß des Steuereffektes von Bedeutung, da eine höhere Wirksamkeit eine geringere Einsatzmenge erlaubt. Da Tonerbindemittel in der Regel eine starke Abhängigkeit der Aufladung von der Aktivierzeit aufweisen, ist es Aufgabe eines Ladungssteuermittels, zum einen Vorzeichen und Höhe der Toneraufladung einzustellen und zum anderen der Aufladungsdrift des Tonerbindemittels entgegenzuwirken und für Konstanz der Toneraufladung zu sorgen.
Ladungssteuermittel, die nicht verhindern können, daß der Toner bzw. Entwickler bei längerer Gebrauchsdauer eine hohe Ladungsdrift zeigt (Alterung), die sogar bewirken kann, daß der Toner bzw. Entwickler eine Ladungsumkehr erfährt, sind daher für die Praxis ungeeignet.
Vollfarbkopierer und Laserdrucker arbeiten nach dem Prinzip der Trichromie, welches eine exakte Farbtonabstimmung der drei Grundfarben (Gelb, Cyan und Magenta) erforderlich macht. Geringste Farbtonverschiebungen auch nur einer der drei Grundfarben verlangt zwingend eine Farbtonverschiebung der beiden anderen Farben, um auch dann originalgetreue Vollfarbkopien bzw. -drucke produzieren zu können.
Wegen dieser in Farbtonern erforderlichen präzisen Abstimmung der Coloristik der einzelnen Farbmittel aufeinander, sind Ladungssteuermittel absolut ohne Eigenfarbe ganz besonders wichtig.

Bei Farbtonern müssen die drei Toner Gelb, Cyan und Magenta neben den genau definierten farblichen Anforderungen auch hinsichtlich ihrer triboelektrischen Eigenschaften exakt aufeinander abgestimmt sein. Diese triboelektrische Abstimmung ist erforderlich, weil beim Vollfarbdruck bzw. bei der Vollfarbkopie aufeinanderfolgend die drei Farbtoner (bzw. vier Farbtoner, wenn Schwarz mit einbezogen wird) im selben Gerät übertragen werden müssen.

Von Farbmitteln ist bekannt, daß sie die triboelektrische Aufladung von Tonern teilweise nachhaltig beeinflussen können (H.-T. Macholdt, A. Sieber, Dyes & Pigments 9 (1988), 119-127; US-PS 4057426). Wegen der unterschiedlichen triboelektrischen Effekte von Farbmitteln und des daraus resultierenden teilweise sehr ausgeprägten Einflusses auf die Toneraufladbarkeit ist es nicht möglich, die Farbmittel in eine einmal erstellte Tonerbasisrezeptur einfach hinzuzufügen. Vielmehr kann es notwendig werden, für jedes Farbmittel eine eigene Rezeptur zu erstellen, für welche z.B. Art und Menge des benötigten Ladungssteuermittels speziell zugeschnitten werden. Dieses Vorgehen ist entsprechend aufwendig und kommt bei Farbtonern für Prozeßfarbe (Trichromie) noch zusätzlich zu den bereits beschriebenen Schwierigkeiten hinzu. Daher sind hochwirksame farblose Ladungssteuermittel erforderlich, die imstande sind, das unterschiedliche triboelektrische Verhalten verschiedener Farbmittel zu kompensieren und dem Toner die gewünschte Aufladung zu verleihen. Auf diese Art und Weise können triboelektrisch sehr unterschiedliche Farbmittel anhand einer einmal erstellten Tonerbasisrezeptur mit ein und demselben Ladungssteuermittel in den verschiedenen erforderlichen Tonern (Gelb, Cyan, Magenta und gegebenenfalls Schwarz) eingesetzt werden.

Darüberhinaus ist für die Praxis wichtig, daß die Ladungssteuermittel eine hohe Thermostabilität und eine gute Dispergierbarkeit besitzen. Typische Einarbeitungstemperaturen für Ladungssteuermittel in die Tonerharze liegen bei Verwendung von z.B. Knetern oder Extrudern zwischen 100 °C und 200 °C. Dementsprechend ist eine Thermostabilität von 200 °C, besser noch 250 °C, von großem Vorteil. Wichtig ist auch, daß die Thermostabilität über einen längeren Zeitraum (ca. 30 Minuten) und in verschiedenen Bindemittelsystemen gewährleistet ist. Dies ist bedeutsam, da immer wieder auftretende Matrixeffekte zum frühzeitigen Zersetzen des Ladungssteuermittels im Tonerharz führen, wodurch eine dunkelgelbe oder dunkelbraune Färbung des Tonerharzes erfolgt und der Ladungssteuereffekt ganz oder teilweise verloren geht. Typische Tonerbindemittel sind Polymerisations-, Polyadditions- und Polykondensationsharze wie z.B. Styrol-, Stryrolacrylat-, Styrolbutadien-, Acrylat-, Polyester-, Phenol- und Epoxidharze, einzeln oder in Kombination, die noch weitere Inhaltsstoffe, wie Farbmittel, Wachse oder Fließhilfsmittel, enthalten können oder im Nachhinein zugesetzt bekommen können.
Für eine gute Dispergierbarkeit ist es von großem Vorteil, wenn das Ladungssteuermittel möglichst keine wachsartigen Eigenschaften, keine Klebrigkeit und einen Schmelz- oder Erweichungspunkt von > 150 °C, besser > 200 °C, aufweist. Eine Klebrigkeit führt häufig zu Problemen beim Zudosieren in die Tonerformulierung, und niedrige Schmelz- bzw. Erweichungspunkte können dazu führen, daß beim Eindispergieren keine homogene Verteilung erreicht wird, da sich das Material z.B. tröpfchenförmig im Trägermaterial zusammenschließt.

Außer in elektrophotographischen Tonern und Entwicklern können Ladungssteuermittel auch zur Verbesserung der elektrostatischen Aufladung von Pulvern und Lacken, insbesondere in triboelektrisch bzw. elektrokinetisch versprühten Pulverlacken, wie sie zur Oberflächenbeschichtung von Gegenständen aus beispielsweise Metall, Holz, Kunststoff, Glas, Keramik, Beton, Textilmaterial, Papier oder Kautschuk zur Anwendung kommen, eingesetzt werden. Die Pulverlacktechnologie kommt u.a. beim Lackieren von Kleingegenständen, wie Gartenmöbeln, Campingartikeln, Haushaltsgeräten, Fahrzeugteilen, Kühlschränken und Regalen, sowie beim Lackieren von kompliziert geformten Werkstücken zur Anwendung. Der Pulverlack bzw. das Pulver erhält seine elektrostatische Aufladung im allgemeinen nach einem der beiden folgenden Verfahren:
a) Beim Corona-Verfahren wird der Pulverlack bzw. das Pulver an einer geladenen Corona vorbeigeführt und hierbei aufgeladen,
b) beim triboelektrischen bzw. elektrokinetischen Verfahren wird vom Prinzip der Reibungselektrizität Gebrauch gemacht.
Der Pulverlack bzw. das Pulver erhält im Sprühgerät eine elektrostatische Aufladung, die der Ladung des Reibungspartners, im allgemeinen ein Schlauch oder Sprührohr (beispielsweise aus Polytetrafluorethylen), entgegengesetzt ist.

Auch eine Kombination von beiden Verfahren ist möglich.

Als Pulverlackharze werden typischerweise Epoxidharze, carboxyl- und hydroxylgruppenhaltige Polyesterharze, Polyurethan- und Acrylharze zusammen mit den entsprechenden Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt.
Typische Härterkomponenten für Epoxidharze sind beispielsweise Säureanhydride, Imidazole sowie Dicyandiamid und deren Abkömmlinge. Für hydroxylgruppenhaltige Polyesterharze sind typische Härterkomponenten beispielsweise Säureanhydride, verkappte Isocyanate, Bisacylurethane, Phenolharze, Melaminharze. Für carboxylgruppenhaltige Polyesterharze sind typische Härterkomponenten beispielsweise Triglycidylisocyanurate oder Epoxidharze. In Acrylharzen kommen als typische Härterkomponenten beispielsweise Oxazoline, Isocyanate, Triglycidylisocyanurate oder Dicarbonsäuren zur Anwendung.
Der Nachteil einer ungenügenden Aufladung ist vor allem bei triboelektrisch bzw. elektrokinetisch versprühten Pulvern und Pulverlacken, die auf Basis von Polyesterharzen, insbesondere carboxylgruppenhaltigen Polyestern, oder auf Basis von sogenannten Mischpulvern, auch Hybridpulver genannt, hergestellt worden sind, zu beobachten. Unter Mischpulvern versteht man Pulverlacke, deren Harzbasis aus einer Kombination von Epoxidharz und carboxylgruppenhaltigem Polyesterharz besteht. Die Mischpulver bilden die Basis für die in der Praxis am häufigsten vertretenen Pulverlacke. Ungenügende Aufladung der oben genannten Pulver und Pulverlacke führt dazu, daß Abscheidequote und Umgriff am zu beschichtenden Werkstück ungenügend sind. (Der Ausdruck "Umgriff" ist ein Maß dafür, inwieweit sich ein Pulver oder Pulverlack am zu beschichtenden Werkstück auch an Rückseiten, Hohlräumen, Spalten und vor allem an Innenkanten und -ecken abscheidet.)

Farblose Ladungssteuermittel werden in zahlreichen Patentschriften beansprucht. Allerdings weisen die bisher bekannten farblosen Ladungssteuermittel eine Reihe von Nachteilen auf, die den Einsatz in der Praxis stark einschränken bzw. zum Teil unmöglich machen. So weisen die in US-PS 4656112 beschriebenen Chrom-, Eisen-, Kobalt- und Zinkkomplexe neben der Schwermetallproblematik auch den Nachteil auf, daß sie teilweise nicht wirklich farblos sind, und somit in Farbtonern bzw. in weißen oder bunten Pulverlacken nur eingeschränkt Anwendung finden können. Die bekannten, an sich geeigneten, quaternären Ammoniumverbindungen sind häufig schwierig zu dispergieren, was zu einer ungleichmäßigen Aufladung des Toners führt. Zudem tritt oft das Problem auf, daß die von diesen Verbindungen erzeugte Tonerladung nicht über einen längeren Aktivierzeitraum (bis zu 24 Stunden Aktivierdauer) hinweg stabil ist, insbesondere bei hoher Temperatur und Luftfeuchtigkeit, was dann im Verlaufe eines Kopier- oder Druckprozesses zur Anreicherung falsch bzw. nicht genügend aufgeladener Tonerteilchen führt und damit den Prozeß zum Erliegen bringt.

Ferner ist bekannt, daß Ladungssteuermittel auf Ammonium- und Immoniumbasis empfindlich gegen Licht oder mechanische Einwirkungen (US-PS 4683188) und thermisch labil sein können, und daß sie Zersetzungsprodukte bilden, die sich nachteilig auf die triboelektrische Aufladung des Toners auswirken können (US-PS 4684596) und/oder eine starke, oft dunkelbraune, Eigenfarbe aufweisen. Darüberhinaus zeigen sie oft wachsartiges Verhalten, zum Teil Wasserlöslichkeit und/oder geringe Wirksamkeit als Ladungssteuermittel. An sich geeignete Ladungssteuermittel auf Basis hochgradig fluorierter Ammonium- und Immoniumverbindungen (DE-OS 3837345) haben den Nachteil einer aufwendigen Synthese, wodurch hohe Herstellungskosten für die entsprechenden Substanzen anfallen, und sind nicht ausreichend thermostabil. Phosphoniumsalze sind als Ladungssteuermittel weniger wirksam als Ammoniumsalze (US-PS 4496643) und können toxikologisch problematisch sein.

In EP-A-0 284 000 werden quartäre Ammoniumsalze beschrieben, die zwei C₈-C₂₂-Alkyl- oder C₈-C₂₂-Alkenylreste besitzen und als ausschließlich positiv aufladbare Ladungssteuermittel für elektrophotographische Toner verwendet werden.

Weitere positiv aufladbare quartäre Ammoniumsalze mit einer C₁₂-C₂₂-Alkylkette und einem Alkylenaryl-Rest im Ammoniumkation und einem organischen Sulfat oder Sulfonat als Anion sind in EP-A-0 053 888 beschrieben.

Die japanischen Offenlegungsschriften Sho-63-23167, Sho-59-78364, Sho-61-147260, Sho-61-258270, Sho-63-226665 offenbaren quartäre Ammoniumsalze mit Alkyl-, Aryl- oder Aralkylresten am Stickstoffatom und als Anionen Carboxylat, Tetraphenylborat, Tetrafluoroborat oder Hexafluorophosphat.

Ladungssteuermittel auf Basis polymerer Ammoniumverbindungen führen zum Teil zu Amingeruch des Toners oder Entwicklers, und die Ladungskontrolleigenschaften dieser Substanzen können sich durch relativ leichte Oxidation und Feuchtigkeitsaufnahme ändern. Des weiteren sind die Oxidationsprodukte gefärbt und daher störend vor allem in Farbtonern.
Die oben genannten Ladungssteuermittel für elektrophotograpische Toner und Entwickler sind z.B. aufgrund ihrer Farbigkeit für den Einsatz in den überwiegend weißen oder klaren triboelektrisch bzw. elektrokinetisch versprühten Pulvern und Pulverlacken nicht geeignet. Des weiteren schränkt mangelnde Thermostabilität den Einsatz derartiger Ladungssteuermittel stark ein, da Pulverlacke beispielsweise bei über 200 °C 15 Minuten lang eingebrannt werden. Die in DE-OS 3837345 beanspruchten Ladungssteuermittel für Pulver und Pulverlacke sind aufgrund von Wachsartigkeit und Wasserlöslichkeit bzw. Hygroskopie schlecht zu handhaben und nur eingeschränkt anwendbar.
Die in der deutschen Patentanmeldung DE-A 40 29 652 beanspruchten Amine als Ladungssteuermittel für Pulver- und Pulverlacke zeigen hinsichtlich der Abscheidequote, die für das Aufbringen des Pulverlacks die entscheidende Größe ist, nur begrenzte Eignung. Weiterhin zeigen sich Schwächen beim Umgriff und in der Dispergierbarkeit.

Ziel der vorliegenden Erfindung ist es daher, verbesserte besonders wirksame farblose Ladungssteuermittel zu finden, wobei neben der Ladungshöhe das schnelle Erreichen und die Konstanz dieser Ladung gewährleistet sein muß, und der Ladungssteuereffekt nicht empfindlich gegenüber Temperatur- und Luftfeuchtigkeits-Veränderungen sein darf. Darüberhinaus sollen diese Verbindungen in hohem Maße thermostabil sein, vor allem auch über einen längeren Zeitraum hinweg im jeweiligen Trägermaterial (Harz), sowie möglichst wasserunlöslich, gut dispergierbar und verträglich mit den Toner- bzw. Pulverlackinhaltsstoffen sein. Des weiteren soll die Synthese der Verbindungen wenig aufwendig und ihre Herstellung kostengünstig sein.

Überraschenderweise hat sich nun gezeigt, daß monomere Diallylammonium-Verbindungen durch gezielte Kombination der Diallylammonium-Kationen mit ausgewählten Anionen besonders hohe und konstante Ladungssteuereigenschaften, sehr gute Thermostabilitäten und Dispergierbarkeiten besitzen.

Gegenstand der vorliegenden Erfindung sind ionogene monomere Diallylammmonium-Verbindungen der allgemeinen Formel (I), 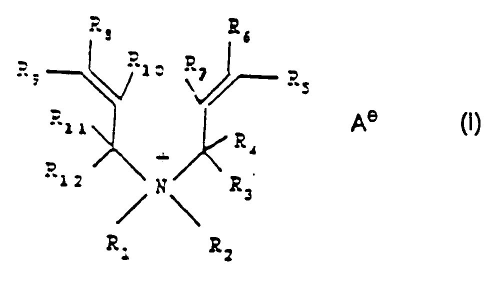 sowie Gemische oder Mischkristalle davon,
wobei die Reste
R₃ bis R₁₂ unabhängig voneinander jeweils ein Wasserstoffatom, Halogenatom, einen Hydroxyl-Rest, einen primären, sekundären oder tertiären Amino-Rest, einen Carbonsäure- oder Carbonsäureester-Rest, einen Acylrest, einen Sulfonsäure- oder Sulfonsäureester-Rest, einen Cyano- oder Nitro-Rest oder jeweils einen Rest auf Basis eines aromatischen Kohlenwasserstoffes, der durch Heteroatome unterbrochen sein kann, bedeuten, R₁ und R₂ die für R₃ bis R₁₂ genannten Bedeutungen haben sowie einen aliphatischen oder aromatischen Kohlenwasserstoffrest, der durch Heteroatome unterbrochen sein kann, bedeuten;
A^{⊖} jeweils das stöchiometrische Äquivalent eines anorganischen Anions aus der Gruppe F⁻, I⁻, OH⁻, HSO₄⁻, S₂⁻, SO₃²⁻, S₂O₃²⁻, HCO₃⁻, CO₃^{2-,} H₂PO₄⁻, HPO₄²⁻, PO₄³⁻, CN⁻, Cyanat, Isocyanat, Zinktetracyanat, Zinktetrathiocyanat, Perchlorat, PF₆⁻, MoO₄²⁻, MoS₄²⁻, WO₄²⁻ oder eine Kombination davon ist, oder eines Anions einer Heteropolysäure oder eines Borats der allgemeinen Formel (II) 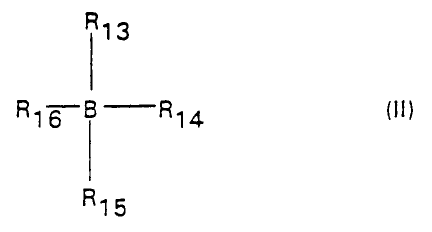 wobei die Reste R₁₃ bis R₁₆ am Borat-Anion unabhängig voneinander aliphatische, cycloaliphatische Reste, Aryl- oder Heteroaryl- oder Aralkylreste, wobei diese Reste durch Alkyl(C₁-C₄)-, Alkoxy(C₁-C₄)-, Arylreste oder Halogenatome substituiert sein können, oder Fluoratome bedeuten,
oder eines organischen Anions, vorzugsweise auf Basis eines Phenolats, olefinischen, aliphatischen oder aromatischen Carboxylats, Thiolats, Sulfonats oder Sulfats, in denen die Alkyl-, Alkenyl- oder Arylreste auch perfluoriert sein können,
oder auf Basis eines Disulfo-pyrrolidinium-betains der allgemeinen Formel (III) 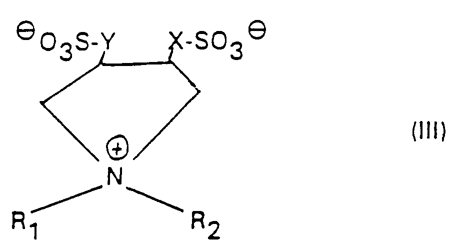 wobei R₁' und R₂' die für R₁ und R₂ genannten Bedeutungen haben sowie X und Y jeweils ein geradkettiger oder verzweigter aliphatischer, gesättigter oder ungesättigter Alkyl(C₁-C₁₈)-Rest oder Alkoxy(C₁-C₁₈)-Rest sind, oder A^{⊖} eine Kombination dieser Anionen bedeutet; sowie Gemische dieser Verbindungen und Mischkristalle mit gemischten Anionen und/oder Kationen, mit Ausnahme der Verbindungen Di(2-propenyl)ammonium-perchlorat, Di(2-propenyl)ammonium-tosylat, Di(2-propenyl)ammoniumfluorid und Di(2-propenyl)ammoniumtetrafluoroborat.
Für die erfindungsgemäße Anwendung sind weiterhin ionogene monomere Diallylammonium-Verbindungen mit Nitrat als Anion von Interesse. Es kommt aber auch eine Kombination dieser Anionen in Frage, insbesondere auch in Kombination mit Cl⁻, Br⁻ oder SO₄²⁻,

Als Anion einer Heteropolysäure werden erfindungsgemäß beispielsweise Molybdatophosphate, wie P (Mo₃O₁₀)₄³⁻, Wolframatophosphate, wie P (W₃O₁₀)₄³⁻, oder Silicomolybdate eingesetzt.

Als Borate werden erfindungsgemäß vorzugsweise Tetrafluoroborat, Tetrachloroborat, Tetraphenylborat, Tetra(fluorphenyl)borat, Tetra(chlorphenyl)borat, Tetratolylborat, Tetranaphthylborat, Tetra(methoxyphenyl)borat, Tetrabiphenylborat, Tetrabenzylborat oder Tetrapyridylborat eingesetzt.

Als organische Anionen A^{⊖} werden erfindungsgemäß beispielsweise Ethylsulfat, Thiolat, Phenolat, Nitrophenolat, gesättigtes oder ungesättigtes aliphatisches oder cycloaliphatisches oder aromatisches Carboxylat oder Sulfonat, vorzugsweise Acetat, Lactat, Benzoat, das Mono- oder Dianion der Dithiodibenzoesäure, Salicylat, 2-Hydroxy-3-naphthoat, 2-Hydroxy-6-naphthoat, Ethylsulfonat, Phenylsulfonat oder Tosylat, ferner perfluoriertes gesättigtes oder ungesättigtes aliphatisches oder cycloaliphatisches oder aromatisches Carboxylat oder Sulfonat, vorzugsweise Perfluoracetat, Perfluor(C₁-C₃₀)alkylbenzoat, Perfluorethylsulfonat oder Perfluor(C₁-C₃₀)alkylbenzolsulfonat, sowie gesättigtes oder ungesättigtes aliphatisches oder cycloaliphatisches oder aromatisches Di- und Tricarboxylat, vorzugsweise Citrat, Oxalat oder Succinat, bzw. Di- und Trisulfonat, chlorierte und fluorierte aliphatische, cycloaliphatische oder aromatische Carboxylate, wie Trifluoracetat und Disulfo-pyrrolidinium-betaine der allgemeinen Formel (III),
wobei R₁' und R₂' gleich R₁ und R₂ sind sowie X und Y geradkettige oder verzweigte aliphatische, gesättigte oder ungesättigte Alkyl(C₁-C₁₈)- oder Alkoxy(C₁-C₁₈)-Reste, bevorzugt Alkyl(C₁-C₅)- oder Alkoxy(C₁-C₅)-Reste, Polyoxalkylen-Reste, bevorzugt Polyoxethylen- und Polyoxpropylen-Reste, der allgemeinen Formel (Alkylen(C₂-C₅)-O)ₙ-R bedeuten, worin R ein Wasserstoffatom oder einen Alkyl(C₁-C₄)-Rest bedeutet und n eine Zahl von 1 bis 10 ist, eingesetzt.

Bevorzugt im Sinne der vorliegenden Erfindung sind Verbindungen der Formel (I), in denen
A^{⊖} ein Anion aus der Gruppe F⁻, J⁻, BF₄⁻, B(Aryl)₄⁻, PF₆⁻, P(Mo₃O₁₀)₄³⁻ oder eines Disulfo-pyrrolidinium-betains der Formel (III) ist;
R₁ und R₂ bzw. R₁' und R₂'
unabhängig voneinander Wasserstoffatome, geradkettige oder verzweigte, gesättigte oder ungesättigte Alkyl(C₁-C₁₈)- oder Alkoxy(C₁-C₁₈)-Reste, Polyoxalkylen-Reste, bevorzugt Polyoxethylen- und/oder Polyoxpropylen-Reste, der allgemeinen Formel (Alkylen(C₁-C₅)-O)ₙ-R, worin R ein Wasserstoffatom, einen Alkyl(C₁-C₄)-Rest oder einen Acyl-Rest, vorzugsweise den Acetyl-, Benzoyl- oder Naphthoyl-Rest, und n eine Zahl von 1 bis 10 ist; Aryl-oder Heteroarylreste, vorzugsweise Phenyl-, Naphthyl- oder Pyridyl-Reste; Aralkyl-Reste, vorzugsweise Tolyl-Reste; Aralkoxy-Reste, vorzugsweise Methoxyphenyl-Reste; Alkaryl-Reste, vorzugsweise Benzyl-Reste; oder Cycloalkyl-Reste, vorzugsweise Cyclopentyl- oder Cyclohexylreste, bedeuten, oder die vorstehend genannten Reste mindestens ein Heteroatom, vorzugsweise Stickstoff, Sauerstoff, Schwefel, Phosphor, oder eine Kombination davon enthalten sowie durch einen oder mehrere Carboxamid-Reste, Sulfonamid-Reste, Urethan-Reste, Keto-Reste, primäre, sekundäre oder tertiäre Amino-Reste, Nitro-Reste, Ether-Reste, insbesondere Alkylen(C₂-C₄)-O-Alkyl(C₁-C₄), Alkyl(C₁-C₄)-Reste, Alkoxy(C₁-C₄)-Reste, Aroxy-Reste, insbesondere Phenoxy-Reste, Halogenalkyl(C₁-C₃₀)-Reste, Halogenalkoxy(C₁-C₃₀)-Reste, Ester-Reste, insbesondere -C(O)O-Alkyl(C₁-C₄), ein oder mehrere Halogenatome, Hydroxyl-, Carboxyl-, Sulfonsäure-, Cyano- oder Mercapto-Gruppen oder eine Kombination davon substituiert sein können,
oder R₁ und R₂ bzw. R₁' und R₂'
zusammen ein gesättigtes oder ungesättigtes, aromatisches oder nichtaromatisches 5- bis 7-gliedriges Ringsystem bilden, vorzugsweise das Pyridinium-Ringsystem, das weitere Heteroatome, bevorzugt Stickstoff, Sauerstoff, Schwefel oder eine Kombination davon, im Ring enthalten kann, insbesondere das Morpholinium-Ringsystem, und das substituiert und/oder durch Ankondensation von oder Verbrückung zu weiteren Ringsystemen modifiziert sein kann, insbesondere das Chinolinium-Ringsystem;
R₃ bis R₁₂
unabhängig voneinander Wasserstoff, Hydroxy, Carboxy, Sulfo, Cyano, Mercapto oder Halogenatome oder Aroxy-Reste, vorzugsweise Phenoxy-Reste, sind;
sowie Gemische dieser Verbindungen und Mischkristalle mit gemischten Anionen und/oder Kationen.

Besonders bevorzugt im Sinne der vorliegenden Erfindung sind Verbindungen der Formel (I), in denen
A^{⊖} ein Anion aus der Gruppe BF₄⁻ oder B(Aryl)₄⁻ ist, worin Aryl Phenyl, Naphthyl, Fluorphenyl, Chlorphenyl, Methoxyphenyl, Biphenyl, Pyridyl oder Tolyl oder eine Kombination davon bedeutet;
R₁ und R₂
Unabhängig voneinander Wasserstoffatome, geradkettige oder verzweigte, gesättigte oder ungesättigte Alkyl(C₁-C₈)- oder Alkoxy(C₁-C₈)-Reste, Aryl- oder Heteroaryl-Reste, insbesondere Phenyl-, Naphthyl- oder Pyridyl-Reste, Aralkyl-Reste, insbesondere Tolyl-Reste, Aralkoxy-Reste, insbesondere Methoxyphenyl-Reste, Alkaryl-Reste, insbesondere Benzyl-Reste, oder Cycloalkyl-Reste, insbesondere Cyclopentyl- oder Cyclohexylreste, bedeuten,
wobei die Reste R₁ und R₂ durch ein oder mehrere Halogenatome, Hydroxyl-, Carboxyl-, Sulfonsäure- oder Carboxamid-Reste, insbesondere -NH-C(O)-Alkyl (C₁-C₄), Sulfonamid-Reste, insbesondere -NH-SO₂-Alkyl(C₁-C₄), Keto-Reste, insbesondere -C(O)-Alkyl(C₁-C₄), primäre, sekundäre oder tertiäre Amino-Reste, insbesondere -NH₂, -NH[Alkyl(C₁-C₄)], -N[Alkyl(C₁-C₄)]₂, Nitro-Reste, Ether-Reste, insbesondere Alkylen(C₂-C₄)-O-Alkyl(C₁-C₄), Alkyl(C₁-C₄)-Reste, Alkoxy (C₁-C₄)-Reste, Aroxy-Reste, insbesondere Phenoxy-Reste, Halogenalkyl(C₁-C₄)-Reste, Halogenalkoxy(C₁-C₄)-Reste oder Ester-Reste, insbesondere -C(O)O-Alkyl(C₁-C₄), substituiert sein können;
R₃ bis R₁₂
unabhängig voneinander Wasserstoff, Hydroxy oder Halogenatome sind sowie Gemische dieser Verbindungen und Mischkristalle mit gemischten Anionen und/oder Kationen.

Ganz besonders bevorzugt im Sinne der vorliegenden Erfindung sind Verbindungen der Typen (1) bis (7) 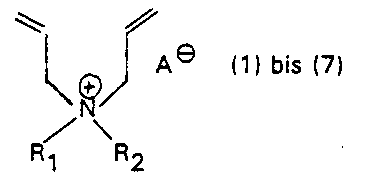 wobei
in der Verbindung (1) A^{⊖} die Bedeutungen BF₄⁻ oder B(Phenyl)₄⁻, R₁ die Bedeutungen H oder CH₃ und R₂ die Bedeutungen CH₃, C₈H₁₇ oder H haben; in der Verbindung (2) A^{⊖} ein Alkyl-3,4-disulfomethyl-pyrrolidinium-betain der Formel (III) bedeutet, wobei X und Y jeweils Alkyl(C₁-C₅) bedeuten und R₁' und R₂' jeweils H oder CH₃ sind sowie beliebige Mischungen oder Mischkristalle der Verbindungstypen (1) und (2); in der Verbindung (3) A^{⊖} die Bedeutung BF₄⁻ hat;
in der Verbindung (4) A^{⊖} die Bedeutung B(C₆H₅)₄⁻ hat,
in der Verbindung (5) A^{⊖} die Bedeutung PF₆⁻ hat,
in der Verbindung (6) A^{⊖} die Bedeutung 1,1 Dialkyl-3,4-disulfomethyl-pyrrolidinium-betain hat sowie
in der Verbindung (7) A^{⊖} die Bedeutung P(Mo₃O₁₀)₄³⁻ hat.

Gegenstand der Erfindung ist weiterhin ein Verfahren zur Herstellung der Verbindungen der Formel (I) durch Anionenaustausch. Die Herstellung erfolgt, indem man ein Diallylammoniumhalogenid der Formel (IV), 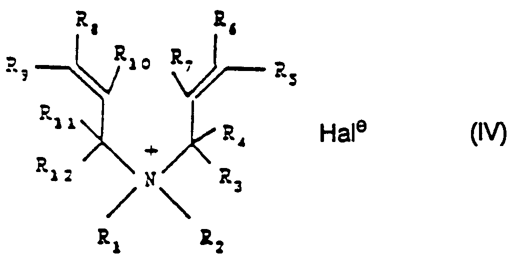 wobei Hal Fluor, Chlor, Brom oder Jod, vorzugsweise Chlor oder Brom, besonders bevorzugt Chlor, bedeutet, gelöst in Wasser oder in einem Gemisch aus Wasser und einem mit Wasser vollständig oder teilweise mischbaren organischen Lösungsmittel, mit einer oder mehreren dem Anion A^{⊖} zugrundeliegenden Verbindung(en), vorzugsweise mit dem Natriumsalz, bei einer Temperatur zwischen 0°C und 100°C, vorzugsweise 10°C und 70°C, und einem pH-Wert zwischen 3 und 10, vorzugsweise 5 und 8, versetzt und anschließend gegebenenfalls die erfindungsgemäße Verbindung der Formel (I) durch Aussalzen mit halogenhaltigen Salzen, beispielsweise Kaliumchlorid, ausfällt. Die erfindungsgemäßen Verbindungen sind in der Regel in Wasser oder im genannten Wasser/Lösemittelgemisch so schwer löslich, daß sie aus der Lösung auch ohne zusätzliches Aussalzen ausfallen.
Die Herstellung der Ausgangsverbindungen, der Diallylammoniumchloride, ist bekannt und in der Literatur ausführlich beschrieben [z.B.Houben-Weyl, "Methoden der Organischen Chemie", Bd. E 20/2, Thieme-Verlag, Stuttgart, 1987, 1023-1028; S.Harada und K. Arai, Makromol.Chem. 107, (1967) 64; Y.Negi, S.Harada und O.Ishizuka, J.Polym.Sci.5,(1967) 64; DE-A-3528985]. Die Verbindungen werden beispielsweise durch Umsetzung von Allylhalogeniden mit N,N-Dialkylallylaminen und Quaternisierung des Diallylamins hergestellt.

Das entsprechende Na-Sulfo-Betain (III) läßt sich nach der DD-A1-224844 gewinnen.

In einer bevorzugten Ausführungform wird der Anionenaustausch in einem Gemisch aus Wasser und Isopropanol, Wasser und Isobutanol oder Wasser und Methylisobutylketon durchgeführt.

In einer weiteren bevorzugten Ausführungsform wird das Diallylammoniumchlorid mit Natriumtetraphenylborat, Natriumtetra-o-fluorphenylborat, Natriumtetra-m-fluorphenylborat, Natriumtetra-p-fluorphenylborat, Natriumtetra-o-chlorphenylborat, Natriumtetra-m-chlorphenylborat, Natriumtetra-p-chlorphenylborat, Natriumtetra-o-tolylborat, Natriumtetra-m-tolylborat, Natriumtetra-p-tolylborat, Natriumtetra-1-naphthylborat, Natriumtetra-2-naphthylborat, Natriumtetra-o-methoxyphenylborat, Natriumtetra-m-methoxyphenylborat, Natriumtetra-p-methoxyphenylborat, Natriumtetra-o-biphenylborat, Natriumtetra-m-biphenylborat, Natriumtetra-p-biphenylborat, Natriumtetrabenzylborat, Natriumtetra-o-pyridylborat, Natriumtetra-m-pyridylborat, Natrium-p-pyridylborat oder Natriumtetrafluoroborat umgesetzt.

Die erfindungsgemäßen Verbindungen sind farblos und besitzen durch gezielte Kombination von bestimmten monomeren Diallylammonium-Kationen mit ausgewählten Anionen besonders hohe und konstante Ladungssteuereigenschaften, sehr gute Thermostabilitäten und Dispergierbarkeiten.
Ein großer technischer Vorteil dieser gut dispergierbaren Verbindungen liegt darin, daß Substanzen derselben Verbindungsklasse je nach Carrier-Harz-Kombination entweder als positives oder negatives Ladungssteuermittel eingesetzt werden können. Es können somit sowohl Positiv- als auch Negativtoner anhand einer festen Tonerbasisrezeptur (bestehend aus Tonerbindemittel, Farbmittel, Fließhilfsmittel und gegebenenfalls weiteren Komponenten), durch Wahl des gewünschten Carriers und/oder Harzes bzw. durch geeignete Kationen/Anionen-Kombination der erfindungsgemäßen Verbindungen hergestellt werden. Im Vergleich zu den polymeren Verbindungen aus der deutschen Patentanmeldung DE-A 4 029 652 zeigen die erfindungsgemäßen monomeren Diallylammonium-Verbindungen eine hohe Kristallinität und sind daher verfahrenstechnisch leichter zu isolieren und darüberhinaus deutlich schneller zu trocknen.
Bei den erfindungsgemäßen Verbindungen sind von ganz besonderem Vorteil die wenig aufwendige Synthese, die im Vergleich zu DE-A 40 29 652 den Schritt der Polymerisation einspart, die kostengünstige Herstellung, die hohe Wirksamkeit, die ausgezeichnete Thermostabilität und insbesondere das Bewirken einer deutlich verbesserten Abscheidequote sowie Umgriffs, so daß insgesamt eine effizientere und ökonomischere Ausnutzung versprühter Pulverlacke resultiert.

Gegenstand der Erfindung ist weiterhin die Verwendung der erfindungsgemäßen Verbindungen sowie der Verbindungen der Formel (I), worin das Anion A^{⊖} zusätzlich zu den aufgeführten Bedeutungen auch Chlorid, Bromid oder Sulfat bedeuten kann, einzeln oder in Kombination, als Ladungssteuermittel in elektrophotographischen Tonern und Entwicklern, die zum Kopieren bzw. Vervielfältigen von Vorlagen sowie zum Drucken von elektronisch, magnetisch oder optisch gespeicherten Informationen oder im Colorproofing eingesetzt werden. Darüberhinaus sind die erfindungsgemäßen Verbindungen geeignet als ladungsverbesserndes Mittel in Pulvern und Lacken zur Oberflächenbeschichtung von Gegenständen aus Metall, Holz, Kunststoff, Glas, Keramik, Beton, Textilmaterial, Papier oder Kautschuk, insbesondere in triboelektrisch bzw. elektrokinetisch versprühten Pulverlacken. Außerdem können diese Verbindungen auch als ladungsverbesserndes Mittel in Form von Beschichtungen von Carriern bzw. Bestandteil von Beschichtungen von Carriern, die in Entwicklern zum elektrophotographischen Kopieren oder Vervielfältigen von Vorlagen sowie zum Drucken von elektronisch, optisch oder magnetisch gespeicherten Informationen oder im Colorproofing zur Anwendung kommen, eingesetzt werden.

Der besondere Vorteil der erfindungsgemäß beanspruchten Verbindungen ist, daß sie farblos sind und einen hohen Ladungssteuereffekt aufweisen und daß dieser über einen längeren Aktivierzeitraum hinweg (bis zu 24 Stunden) konstant ist.

So zeigt z. B. ein Test-Toner mit 1 Gewichtsprozent der Verbindung des Typs (4) nach 10 Minuten eine Aufladung von -19 µC/g, nach 30 Minuten von -18 µC/g, nach 2 Stunden von -15 µC/g und nach 24 Stunden von -15 µC/g (Anwendungsbeispiel 1).

Ein Test-Pulverlack mit 1 Gewichtsprozent der Verbindung (4) zeigt nach 10 Minuten eine Aufladung von +3 µC/g, +4 µC/g nach 30 Minuten, +7 µC/g nach 2 Stunden und +6 µC/g nach 24 Stunden (Anwendungsbeispiel 5).

Der hohe Ladungssteuereffekt wird um so deutlicher, wenn zum Vergleich das Aufladungsverhalten des reinen Tonerbindemittels, z.B. ®Dialec S-309, betrachtet wird: (Vergleichsbeispiel 1): -4 µC/g nach 10 Minuten, -12 µC/g nach 30 Minuten,
-27 µC/g nach 2 Stunden, -48 µC/g nach 24 Stunden; bzw. eines reinen Pulverlackbindemittels, z.B. ®Crylcoat 430, (Vergleichsbeispiel 2):
-20 µC/g nach 10 Minuten, -15 µC/g nach 30 Minuten, -8 µC/g nach 2 Stunden, -4 µC/g nach 24 Stunden.

Für die Praxis von großer Bedeutung ist, daß die erfindungsgemäßen Verbindungen chemisch inert und gut verträglich mit Bindemitteln, beispielsweise Styrolacrylaten, Polyestern, Epoxiden und Polyurethanen, sind. Zudem sind die Verbindungen thermostabil und können somit ohne Schwierigkeiten mit den gängigen Verfahren (Extrudieren, Kneten) unter den üblichen Bedingungen (Temperaturen zwischen 100 °C und 200 °C) in die gängigen Bindemittel eingearbeitet werden. Die Synthese der erfindungsgemäßen Verbindungen ist wenig aufwendig, und die Produkte fallen in hoher Reinheit an.

Die erfindungsgemäß verwendeten Verbindungen werden in der Regel in einer Konzentration von etwa 0,01 bis etwa 30 Gewichtsprozent, vorzugsweise von etwa 0,1 bis 5,0 Gewichtsprozent, in das Bindemittel des jeweiligen Toners oder Entwicklers oder Lacks oder Pulverlacks homogen, beispielsweise durch Extrudieren oder Einkneten, eingearbeitet. Dabei können die Ladungssteuermittel für Toner bzw. ladungsverbessernde Mittel für Pulver und Lacke zur Oberflächenbeschichtung, insbesondere für triboelektrisch bzw. elektrokinetisch versprühte Pulverlacke, als getrocknete und gemahlene Pulver, Dispersionen oder Lösungen, Preßkuchen, Masterbatch, als auf geeignete Träger, wie z.B. Kieselgel, TiO₂, Al₂O₃, aus wäßriger oder nichtwäßriger Lösung aufgezogene Verbindungen oder in sonstiger Form zugegeben werden. Ebenso können die erfindungsgemäß eingesetzten Verbindungen grundsätzlich auch schon bei der Herstellung der jeweiligen Bindemittel zugegeben werden, d.h. im Verlauf von deren Polymerisation, Polyaddition oder Polykondensation.

Die Höhe der elektrostatischen Aufladung der elektrophotographischen Toner bzw. der Pulverlacke, in welche die erfindungsgemäßen Ladungssteuermittel homogen eingearbeitet wurden, wurde an Standardtestsystemen unter gleichen Bedingungen (wie gleiche Dispergierzeiten, gleiche Teilchengrößenverteilung, gleiche Teilchenform) bei etwa 20°C und 50 % relativer Luftfeuchte gemessen. Die elektrostatische Aufladung des Toners bzw. Pulverlackes erfolgte durch Verwirbelung mit einem Carrier, d.h. einem standardisiertem Reibungspartner (3 Gewichtsteile Toner auf 97 Gewichtsteile Carrier), auf einer Rollbank (150 Umdrehungen pro Minute). Anschließend wurde an einem üblichen q/m-Meßstand die elektrostatische Aufladung gemessen (J.H. Dessauer, H.E. Clark, "Xerography and related Processes", Focal Press, N. Y., 1965, Seite 289; J.F. Hughes, "Electrostatic Powder Coating", Research Studies Press Ltd., Letchworth, Hertfordshire, England, 1984, Chapter 2). Bei der Bestimmung des q/m-Wertes ist die Teilchengröße von großem Einfluß, weshalb bei den durch Sichtung erhaltenen Toner- bzw. Pulverlackproben streng auf einheitliche Teilchengrößenverteilung geachtet wurde. So wird für Toner eine mittlere Teilchengröße von 10 µm angestrebt, während für Pulverlacke eine mittlere Teilchengröße von 50 µm praktikabel ist.

Die Triboversprühung der Pulver(lacke) wurde mit einem Sprühgerät, beispielsweise ®Tribo Star der Firma Intec (Dortmund), mit einem Normsprührohr und einer Sterninnenstange bei maximalem Pulverdurchsatz mit einem Sprühdruck von 3 bar durchgeführt. Der zu besprühende Gegenstand wurde hierzu in einer Spritzkabine aufgehängt und aus ca. 20 cm Abstand direkt von vorne ohne weitere Bewegung des Sprühgerätes besprüht. Die jeweilige Aufladung des versprühten Pulvers wurde anschließend mit einem "Meßgerät zur Messung von triboelektrischer Ladung von Pulvern" der Firma Intec (Dortmund) gemessen. Zur Messung wurde die Meßantenne des Meßgerätes direkt in die aus dem Sprühgerät austretende Pulverwolke gehalten. Die aus der elektrostatischen Ladung von Pulverlack oder Pulver sich ergebende Stromstärke wurde in µA angezeigt. Die Abscheidequote wurde anschließend in % durch eine Differenzwiegung aus versprühtem und abgeschiedenem Pulverlack bestimmt.

Die nachstehenden Beispiele dienen zur Erläuterung der Erfindung, ohne diese darauf zu beschränken. Die Angabe "Min." bedeutet "Minuten" und "Std." bedeutet "Stunden".

### Herstellungsbeispiel 1

### Verbindung (4)

67,36 g (0,25 Mol) einer 60 gew.-%igen wäßrigen Diallylammoniumchlorid-Lösung wurden in 1000 ml entionisiertem Wasser eingetragen. Zu der Lösung wurden bei 50 bis 60°C 92,24 g (0,25 Mol) NaB (C₆H₅)₄ innerhalb von 3 Min. unter intensivem Rühren zugegeben. Es trat eine voluminöse, weiße Fällung auf. Die Suspension wurde 10 Min. bei 50°C nachgerührt, auf etwa 20°C abgekühlt, abgesaugt und mehrmals intensiv nachgewaschen. Abschließend wurde die Substanz im Vakuum bei 200 mbar und 90°C getrocknet.
- Ausbeute:: 111.07 g (99 % der Theorie)
- Zersetzung:: 240°C

| | | | | | |
|---|---|---|---|---|---|
| Elementaranalyse: | C 86,0 %; | H 8,0 %; | N 3,2 %; | B 2,6 % | (gefunden) |
| | C 86,6 %; | H 7,9 %; | N 3,1 %; | B 3,0 % | (berechnet). |

### Herstellungsbeispiel 2

### Verbindung (7)

40,0 g (0,15 Mol) einer 60 gew.-%igen wäßrigen Diallylammoniumchlorid-Lösung wurden bei etwa 20°C mit einer gesättigten Lösung von 94,5 g (0.045 Mol) Na₃P[Mo₃O₁₀]₄ in 210 ml Wasser versetzt. Die entstehende Suspension wurde 10 Min. nachgerührt, abgesaugt und mit kaltem Wasser chloridfrei gewaschen. Abschließend wurde die Substanz im Vakuum bei 200 mbar und 90°C getrocknet.
- Ausbeute:: 48,6 g (43,5 % der Theorie)
- Zersetzung: > 300°C

| | | | | | |
|---|---|---|---|---|---|
| Elementaranalyse: | C 12,1 %; | H 2,5 %; | N 2,1 %; | P 1,5 % | (gefunden) |
| | C 11,2 %; | H 2,7 %; | N 1,6 %; | P 1,2 % | (berechnet). |

### Herstellungsbeispiel 3

### Verbindung (5)

40,0 g (0,15 Mol) einer 60 gew.-%igen wäßrigen Diallylammoniumchlorid-Lösung wurden bei 50°C mit einer etwa 50°C warmen Lösung von 27,4 g (0,15 Mol) NaPF₆ in 150 ml entionisiertem Wasser versetzt und auf 80°C erwärmt. Nach 15 Min. ließ man abkühlen und verdoppelte das Volumen durch Zugabe von 200 ml einer wäßrigen 1 normalen KCl-Lösung. Nach dem Abkühlen auf 0°C wurde abgesaugt und mit kaltem Wasser chloridfrei gewaschen. Abschließend wurde die Substanz im Vakuum bei 200 mbar und 90°C getrocknet.
- Ausbeute:: 14.1 g (50 % der Theorie)
- Zersetzung: > 260°C

| | | | | | | |
|---|---|---|---|---|---|---|
| Elementaranalyse: | C 35,1 %; | H 5,6 %; | N 5,0 %; | P 11,3 % | | (gefunden) |
| | C 35,4 %; | H 5,9 %; | N 5,1 %; | P 11,4 %; | F 42,0 % | (berechnet). |

### Anwendungsbeispiel 1

1,0 Teile der Verbindung (4) wurden mittels eines Kneters 45 Minuten in 99,0 Teile Tonerbindemittel ("Dialec S-309" der Firma Diamond Shamrock, Styrol-Methacrylat-Copolymer 60:40) homogen eingearbeitet. Anschließend wurde auf einer Laboruniversalmühle gemahlen und dann auf einem Zentrifugalsichter klassifiziert. Die gewünschte Teilchenfraktion (4 bis 25 µm) wurde mit einem Carrier aus mit Styrol-Methacrylat-Copolymer 90:10 beschichteten Magnetit-Teilchen der Größe 50 bis 200 µm des Typs "90 µm Xerographic Carrier" der Firma Plasma Materials Inc. (Manchester, NH, USA) aktiviert.

Die Messung erfolgte an einem üblichen q/m-Meßstand. Durch Verwendung eines Siebes mit einer Maschenweite von 25 µm wurde sichergestellt, daß bei den Tonerausblasungen kein Carrier mitgerissen werden konnte. Die Messungen erfolgten bei etwa 20°C und 50 % relativer Luftfeuchte. In Abhängigkeit von der Aktivierdauer wurden folgende q/m-Werte [µC/g] gemessen:

| Aktivierdauer | Aufladung q/m [µC/g] |
|---|---|
| 10 Min. | -19 |
| 30 Min. | -18 |
| 2 Std. | -15 |
| 24 Std. | -15 |

### Anwendungsbeispiele 2 und 3

Jeweils 1 Teil der Verbindungen (5) und (7), entsprechend Anwendungsbeispielen 2 und 3 wurden, wie in Anwendungsbeispiel 1 beschrieben, in jeweils 99 Teile Tonerbindemittel homogen eingearbeitet. In Abhängigkeit von der Aktivierdauer wurden folgende q/m-Werte [µC/g] gemessen:

| Aktivierdauer | Aufladung q/m [µC/g] | |
|---|---|---|
| | Anwendungsbeispiel 2 | 3 |
| 10 Min. | +3 | -5 |
| 30 Min. | +5 | -10 |
| 2 Std. | +2 | -16 |
| 24 Std. | +2 | -20 |

### Anwendungsbeispiel 4

1 Teil der Verbindung (4) wurde, wie in Anwendungsbeispiel 1 beschrieben, in 99 Teile eines Pulverlackbindemittels auf Basis eines carboxylhaltigen Polyesterharzes, z.B. ®Crylcoat 430 der Firma UCB/Belgien, homogen eingearbeitet. In Abhängigkeit von der Aktivierdauer wurden folgende q/m-Werte [µC/g] gemessen, wobei an Stelle des in Anwendungsbeispiel 1 beschriebenen Styrolacrylat-beschichteten Carriers ein mit PTFE (Polytetrafluorethylen) beschichteter Carrier verwendet wurde:

| Aktivierdauer | q/m [µC/g] |
|---|---|
| 10 Min. | +3 |
| 30 Min. | +4 |
| 2 Std. | +7 |
| 24 Std. | +6 |

Zur Bestimmung der Abscheidequote wurden 30 g des Test-Pulverlackes mit einem definierten Druck durch eine wie zuvor beschriebene Tribopistole versprüht. Durch Differenzwiegung ließ sich die abgeschiedene Pulverlackmenge bestimmen und eine Abscheidequote in % definieren sowie durch die Ladungsübertragung ein Stromfluß (µA) abgreifen.

| Druck [bar] | Strom [µA] | Abscheidequote [%] |
|---|---|---|
| 3 | 1,4-1,8 | 52 |

### Vergleichsbeispiel 1

Zur Messung des reinen Harz-Bindemittels Dialec S 309 wurde wie in Anwendungsbeispiel 1 verfahren, ohne jedoch Zusatzstoffe einzukneten.

| Aktivierdauer | q/m [µC/g] |
|---|---|
| 10 Min. | -4 |
| 30 Min. | -12 |
| 2 Std. | -27 |
| 24 Std. | -48 |

### Vergleichsbeispiel 2

Zur Bestimmung des Aufladeverhaltens der reinen Pulverlackharz-Komponente Crylcoat 430 wurde wie in Vergleichsbeispiel 1 verfahren, wobei an Stelle des Magnetit-Carriers ein mit PTFE (Polytetrafluorethylen) gecoateter Carrier verwendet wurde.

| Aktivierdauer | q/m [µC/G] |
|---|---|
| 10 Min. | -17 |
| 30 Min. | -19 |
| 2 Std. | -19 |
| 24 Std. | -18 |

Zur Bestimmung der Abscheidequote wurde wie in Anwendungsbeispiel 5 verfahren.

| Druck [bar] | Strom [µA] | Abscheidequote [%] |
|---|---|---|
| 3 | 0.1 | 5 |

### Vergleichsbeispiel 3

Zur Bestimmung des triboelektrischen Aufladeverhaltens einer vergleichbaren polymeren ionogenen Ammonium-Verbindung wurde wie in Anwendungsbeispiel 5 verfahren:

| Aktivierdauer | q/m [µC/g] |
|---|---|
| 10 Min. | - 8 |
| 30 Min. | - 8 |
| 2 Std. | - 9 |
| 24 Std. | -10 |

Zur Bestimmung der Kenndaten bei triboelektrischer Pulverlackversprühung einer vergleichbaren polymeren Ammonium-Verbindung wurde wie in Anwendungsbeispiel 5 verfahren.

| Druck [bar] | Strom [µA] | Abscheidequote [%] |
|---|---|---|
| 3 | 0,7 - 0,9 | 18 |

Ein Vergleich sowohl mit der reinen Pulverlackharz-Komponente (Vergleichsbeispiel 2) als auch mit einer vergleichbaren polymeren Ammonium-Verbindung (Vergleichsbeispiel 3; die Herstellung der eingesetzten polymeren Ammonium-Verbindung ist in DE-A-4029 652, Herstellungsbeispiel 2, beschrieben) zeigt signifikant eine deutliche Verbesserung der Abscheidequote durch die Verwendung des erfindungsgemäßen ionogenen Diallylammonium-Monomeren.

## Patentansprüche

1. Ionogene monomere Diallylammonium-Verbindung der allgemeinen Formel (I) 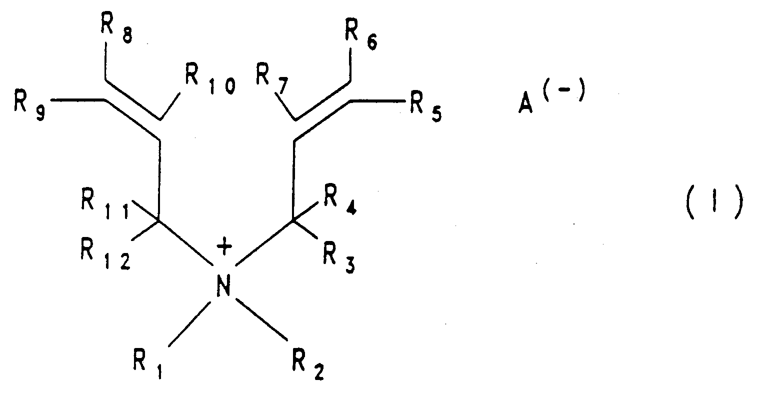 sowie Gemische oder Mischkristalle davon, wobei die Reste
R₃ bis R₁₂ unabhängig voneinander jeweils ein Wasserstoffatom, Halogenatom, einen Hydroxyl-Rest, einen primären, sekundären oder tertiären Amino-Rest, einen Carbonsäure- oder Carbonsäureester-Rest, einen Acylrest, einen Sulfonsäure- oder Sulfonsäureester-Rest, einen Cyano- oder Nitro-Rest oder jeweils einen Rest auf Basis eines aromatischen Kohlenwasserstoffes, der durch Heteroatome unterbrochen sein kann, bedeuten;
R₁ und R₂ die für R₃ bis R₁₂ genannten Bedeutungen haben sowie einen aliphatischen oder aromatischen Kohlenwasserstoffrest, der durch Heteroatome unterbrochen sein kann, bedeuten;
A^{⊖} jeweils das stöchiometrische Aquivalent eines anorganischen Anions aus der Gruppe F⁻, J⁻, OH⁻, HSO₄⁻, S₂⁻, SO₃²⁻, S₂O₃²⁻, HCO₃⁻, CO₃²⁻, H₂PO₄⁻, HPO₄²⁻, PO₄³⁻, CN⁻, Cyanat, Isocyanat, Zinktetracyanat, Zinktetrathiocyanat, Perchlorat, PF₆⁻, MoO₄²⁻, MoS₄²⁻, WO₄²⁻ oder eine Kombination davon ist, oder eines Anions einer Heteropolysäure oder eines Borats der allgemeinen Formel (II) 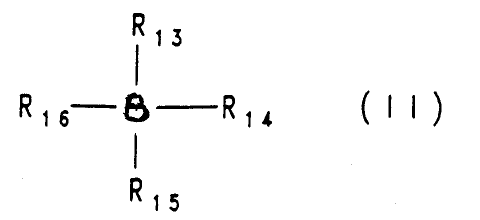 wobei die Reste R₁₃ bis R₁₆ am Borat-Anion unabhängig voneinander aliphatische, cycloaliphatische Reste, Aryl- oder Heteroaryl- oder Aralkylreste, wobei diese Reste durch Alkyl(C₁-C₄)-, Alkoxy(C₁-C₄)-, Arylreste oder Halogenatome substituiert sein können, oder Fluoratome bedeuten, oder eines organischen Anions auf Basis eines Phenolats, olefinischen, aliphatischen oder aromatischen Carboxylats, Sulfonats, Thiolats oder Sulfats, in denen die Alkyl-, Alkenyl- oder Arylreste auch perfluoriert sein können, oder auf Basis eines Disulfo-pyrrolidinium-betains der allgemeinen Formel (III), 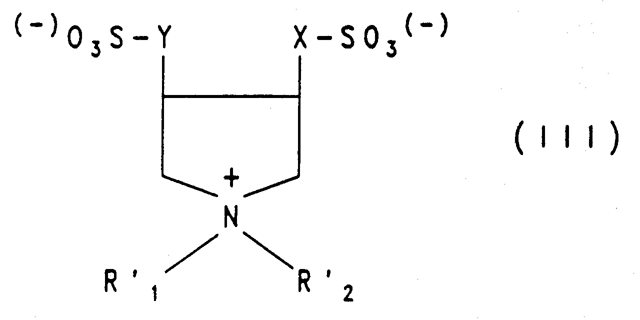 wobei R₁' und R₂' die für R₁ und R₂ genannten Bedeutungen haben sowie X und Y jeweils ein geradkettiger oder verzweigter aliphatischer, gesättigter oder ungesättigter Alkyl(C₁-C₁₈)-Rest oder Alkoxy(C₁-C₁₈)-Rest ist, sowie Gemische dieser Verbindungen und Mischkristalle mit gemischten Anionen und/oder Kationen, mit Ausnahme der Verbindungen Di(2-propenyl)ammonium-perchlorat, Di(2-propenyl)ammoniumtosylat, Di(2-propenyl)ammoniumfluorid und Di(2-propenyl)ammoniumtetrafluoroborat.

2. Verbindung nach Anspruch 1, dadurch gekennzeichnet, daß das Anion A^{⊖} eine Kombination aus den genannten Anionen mit Cl⁻, Br⁻ und/oder SO₄²⁻ist.

3. Verbindung nach Anspruch 1, dadurch gekennzeichnet, daß das Anion A^{⊖} ein Anion einer Heteropolysäure ist, vorzugsweise P(Mo₃O₁₀)₄³⁻, P(W₃O₁₀)₄³⁻ oder ein Silicomolybdat.

4. Verbindung nach Anspruch 1, dadurch gekennzeichnet, daß das Anion A^{⊖} Tetrafluoroborat, Tetrachloroborat, Tetraphenylborat, Tetra(fluorphenyl)borat, Tetra(chlorphenyl)borat, Tetratolyborat, Tetranaphthylborat, Tetra(methoxyphenyl)borat, Tetrabiphenylborat, Tetrabenzylborat oder Tetrapyridylborat oder eine Kombination davon ist.

5. Verbindung nach Anspruch 1, dadurch gekennzeichnet, daß das Anion A^{⊖} ein organisches Anion aus der Gruppe Ethylsulfat, Phenolat, Nitrophenolat, Thiolat, Acetat, Lactat, Benzoat, das Mono- oder Dianion der Dithiodibenzoesäure, Salicylat, 2-Hydroxy-3-naphthoat, 2-Hydroxy-6-naphthoat, Ethylsulfonat, Phenylsulfonat, Tosylat, Perfluoracetat, Perfluor-C₁-C₃₀-alkylbenzoat, Perfluorethylsulfonat, Perfluor-C₁-C₃₀-alkylbenzolsulfonat, gesättigtes oder ungesättigtes aliphatisches oder cycloaliphatisches oder aromatisches Di- und Tricarboxylat oder Di- oder Trisulfonat, vorzugsweise Citrat, Oxalat oder Succinat, ist.

6. Verbindung nach Anspruch 1, dadurch gekennzeichnet, daß das Anion A^{⊖} ein Disulfo-pyrrolidinium-betain der Formel (III) ist, wobei R₁' und R₂' gleich R₁ und R₂ sind sowie X und Y geradkettige oder verzweigte aliphatische, gesättigte oder ungesättigte Alkyl(C₁-C₁₈)- oder Alkoxy(C₁-C₁₈)-Reste, bevorzugt Alkyl(C₁-C₅)- oder Alkoxy(C₁-C₅)-Reste, Polyoxalkylen-Reste, bevorzugt Polyoxethylen- und Polyoxpropylen-Reste, der allgemeinen Formel (Alkylen(C₂-C₅)-O)ₙ-R bedeuten, worin R ein Wasserstoffatom oder einen Alkyl(C₁-C₄)-Rest bedeutet und n eine Zahl von 1 bis 10 ist.

7. Verbindung nach Anspruch 1, dadurch gekennzeichnet, daß das Anion A^{⊖} ein Anion aus der Gruppe F⁻, J⁻, BF₄⁻, B(Aryl)₄⁻, PF₆⁻, P(Mo₃O₁₀)₄³⁻ oder eines Disulfo-pyrrolidinium-betains der Formel (III) ist;
R₁ und R₂ bzw. R₁' und R₂'
unabhängig voneinander Wasserstoffatome, geradkettige oder verzweigte, gesättigte oder ungesättigte Alkyl(C₁-C₁₈)- oder Alkoxy(C₁-C₁₈)-Reste, Polyoxalkylen-Reste, bevorzugt Polyoxethylen- und/oder Polyoxpropylen-Reste, der allgemeinen Formel (Alkylen(C₁-C₅)-O)ₙ-R, worin R ein Wasserstoffatom, einen Alkyl(C₁-C₄)-Rest oder einen Acyl-Rest, vorzugsweise den Acetyl-, Benzoyl- oder Naphthoyl-Rest, und n eine Zahl von 1 bis 10 ist; Aryl-oder Heteroarylreste, vorzugsweise Phenyl-, Naphthyl- oder Pyridyl-Reste; Aralkyl-Reste, vorzugsweise Tolyl-Reste; Aralkoxy-Reste, vorzugsweise Methoxyphenyl-Reste; Alkaryl-Reste, vorzugsweise Benzyl-Reste; oder Cycloalkyl-Reste, vorzugsweise Cyclopentyl- oder Cyclohexylreste, bedeuten, oder die vorstehend genannten Reste mindestens ein Heteroatom, vorzugsweise Stickstoff, Sauerstoff, Schwefel, Phosphor, oder eine Kombination davon enthalten sowie durch einen oder mehrere Carboxamid-Reste, Sulfonamid-Reste, Urethan-Reste, Keto-Reste, primäre, sekundäre oder tertiäre Amino-Reste, Nitro-Reste, Ether-Reste, insbesondere Alkylen(C₂-C₄)-O-Alkyl(C₁-C₄), Alkyl(C₁-C₄)-Reste, Alkoxy(C₁-C₄)-Reste, Aroxy-Reste, insbesondere Phenoxy-Reste, Halogenalkyl(C₁-C₃₀)-Reste, Halogenalkoxy(C₁-C₃₀)-Reste, Ester-Reste, insbesondere -C(O)O-Alkyl(C₁-C₄), ein oder mehrere Halogenatome, Hydroxyl-, Carboxyl-, Sulfonsäure-, Cyano- oder Mercapto-Gruppen oder eine Kombination davon substituiert sein können,
oder R₁ und R₂ bzw. R₁' und R₂'
zusammen ein gesättigtes oder ungesättigtes, aromatisches oder nichtaromatisches 5- bis 7-gliedriges Ringsystem bilden, vorzugsweise das Pyridinium-Ringsystem, das weitere Heteroatome, bevorzugt Stickstoff, Sauerstoff, Schwefel oder eine Kombination davon, im Ring enthalten kann, insbesondere das Morpholinium-Ringsystem, und das substituiert und/oder durch Ankondensation von oder Verbrückung zu weiteren Ringsystemen modifiziert sein kann, insbesondere das Chinolinium-Ringsystem; und
R₃ bis R₁₂
unabhängig voneinander Wasserstoff, Hydroxy, Carboxy, Sulfo, Cyano, Mercapto, Halogenatome oder Aroxy-Reste, vorzugsweise Phenoxy-Reste, sind.

8. Verbindung nach Anspruch 1 oder 7, dadurch gekennzeichnet, daß A^{⊖} ein Anion aus der Gruppe BF₄⁻ oder B(Aryl)₄₋ ist, worin Aryl Phenyl, Naphthyl, Fluorphenyl, Chlorphenyl, Methoxyphenyl, Biphenyl, Pyridyl oder Tolyl oder eine Kombination davon bedeutet;
R₁ und R₂
unabhängig voneinander Wasserstoffatome, geradkettige oder verzweigte, gesättigte oder ungesättigte Alkyl(C₁-C₈)- oder Alkoxy(C₁-C₈)-Reste, Aryl- oder Heteroaryl-Reste, insbesondere Phenyl-, Naphthyl- oder Pyridyl-Reste, Aralkyl-Reste, insbesondere Tolyl-Reste, Aralkoxy-Reste, insbesondere Methoxyphenyl-Reste, Alkaryl-Reste, insbesondere Benzyl-Reste, oder Cycloalkyl-Reste, insbesondere Cyclopentyl- oder Cyclohexylreste, bedeuten,
wobei die Reste R₁ und R₂ durch ein oder mehrere Halogenatome, Hydroxyl-, Carboxyl-, Sulfonsäure- oder Carboxamid-Reste, insbesondere -NH-C(O)-Alkyl-(C₁-C₄), Sulfonamid-Reste, insbesondere -NH-SO₂-Alkyl(C₁-C₄), Keto-Reste, insbesondere -C(O)-Alkyl(C₁-C₄), primäre, sekundäre oder tertiäre Amino-Reste, insbesondere -NH₂, -NH[Alkyl(C₁-C₄)], -N[Alkyl(C₁-C₄)]₂, Nitro-Reste, Ether-Reste, insbesondere Alkylen(C₂-C₄)-O-Alkyl(C₁-C₄), Alkyl(C₁-C₄)-Reste, Alkoxy (C₁-C₄)-Reste, Aroxy-Reste, insbesondere Phenoxy-Reste, Halogenalkyl(C₁-C₄)-Reste, Halogenalkoxy(C₁-C₄)-Reste oder Ester-Reste, insbesondere -C(O)O-Alkyl(C₁-C₄), substituiert sein können;
R₃ bis R₁₂
unabhängig voneinander Wasserstoff, Hydroxy oder Halogenatome sind.

9. Verbindung nach mindestens einem der Ansprüche 1, 4 und 6 bis 8, dadurch gekennzeichnet, daß A^{⊖} die Bedeutung BF₄⁻, B(Phenyl)₄⁻ oder Disulfopyrrolidinium-betain der Formel (III) hat, wobei R₁' und R₂' die nachstehend für R₁ und R₂ angegebenen Bedeutungen besitzen, R₁ Wasserstoff oder Methyl bedeutet, R₂ Wasserstoff, Methyl oder Octyl ist und R₃ bis R₁₂ jeweils Wasserstoff bedeuten.

10. Mischkristall oder Gemisch von zwei oder mehreren Verbindungen gemäß mindestens einem der Ansprüche 1 bis 11.

11. Verfahren zur Herstellung einer Verbindung nach mindestens einem der Ansprüche 1 bis 10, dadurch gekennzeichnet, daß man ein Diallylammoniumhalogenid der Formel (IV), 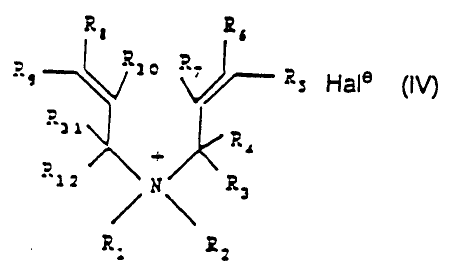 wobei Hal Fluor, Chlor, Brom oder Jod bedeutet, gelöst in Wasser oder in einem Gemisch aus Wasser und einem mit Wasser vollständig oder teilweise mischbaren organischen Lösungsmittel, mit einer oder mehreren dem Anion A^{⊖} zugrundeliegenden Verbindung(en), vorzugsweise mit dem Natriumsalz, bei einer Temperatur zwischen 0°C und 100°C, vorzugsweise 10°C und 70°C, und einem pH-Wert zwischen 3 und 10, vorzugsweise 5 und 8, versetzt und anschließend gegebenenfalls die erfindungsgemäße Verbindung der Formel (I) durch Aussalzen mit halogenhaltigen Salzen, beispielsweise Kaliumchlorid, ausfällt.

12. Verfahren nach Anspruch 11, dadurch gekennzeichnet, daß man in einem Gemisch aus Wasser und Isopropanol, Wasser und Isobutanol oder Wasser und Methylisobutylketon umsetzt.

13. Verfahren nach Anspruch 11 oder 12, dadurch gekennzeichnet, daß das Diallylammoniumchlorid mit Natriumtetraphenylborat, Natriumtetra-o-fluorphenylborat, Natriumtetra-m-fluorphenylborat, Natriumtetra-p-fluorphenylborat, Natriumtetra-o-chlorphenylborat, Natriumtetra-m-chlorphenylborat, Natriumtetra-p-chlorphenylborat, Natriumtetra-o-tolylborat, Natriumtetra-m-tolylborat, Natriumtetra-p-tolylborat, Natriumtetra-1-naphthylborat, Natriumtetra-2-naphthylborat, Natriumtetra-o-methoxyphenylborat, Natriumtetra-m-methoxyphenylborat, Natriumtetra-p-methoxyphenylborat, Natriumtetra-o-biphenylborat, Natriumtetra-m-biphenylborat, Natriumtetra-p-biphenylborat, Natriumtetrabenzylborat, Natriumtetra-o-pyridylborat, Natriumtetra-m-pyridylborat, Natrium-p-pyridylborat oder Natriumtetrafluoroborat umgesetzt wird.

14. Verwendung einer Verbindung nach mindestens einem der Ansprüche 1 bis 10 als Ladungssteuermittel in elektrophotographischen Tonern und Entwicklern, die zum Kopieren bzw. Vervielfältigen von Vorlagen sowie zum Drucken von elektronisch, magnetisch oder optisch gespeicherten Informationen oder im Colorproofing eingesetzt werden, sowie als Ladungssteuermittel in Pulvern und Pulverlacken.

15. Verwendung nach Anspruch 14, dadurch gekennzeichnet, daß man die ionogene monomere Diallylammonium-Verbindung einzeln oder in Kombination in einer Konzentration von etwa 0,01 bis etwa 30, vorzugsweise 0,1 bis 5,0, Gewichtsprozent einsetzt.

16. Verwendung einer Verbindung nach mindestens einem der Ansprüche 1 bis 10, dadurch gekennzeichnet, daß diese Verbindungen als ladungsverbesserndes Mittel in Form von Beschichtungen von Carriern oder Bestandteil von Beschichtungen von Carriern, die in Entwicklern zum elektrophotographischen Kopieren oder Vervielfältigen von Vorlagen sowie zum Drucken von elektronisch, optisch oder magnetisch gespeicherten Informationen oder im Colorproofing zur Anwendung kommen, eingesetzt werden.

17. Verwendung einer Verbindung nach mindestens einem der Ansprüche 1 bis 10, dadurch gekennzeichnet, daß diese Verbindungen einzeln oder In Kombination als ladungsverbesserndes Mittel in Pulvern und Lacken zur Oberflächenbeschichtung von Gegenständen aus Metall, Holz, Kunststoff, Glas, Keramik, Beton, Textilmaterial, Papier oder Kautschuk, insbesondere in triboelektrisch oder elektrokinetisch versprühten Pulverlacken, eingesetzt werden.

18. Verwendung nach mindestens einem der Ansprüche 14 bis 17, dadurch gekennzeichnet, daß das Anion A^{⊖} in der Verbindung der Formel (I) Chlorid, Bromid oder das stöchiometrische Äquivalent von Sulfat ist.

## Claims

1. An ionic monomeric diallylammonium compound of the formula (I) 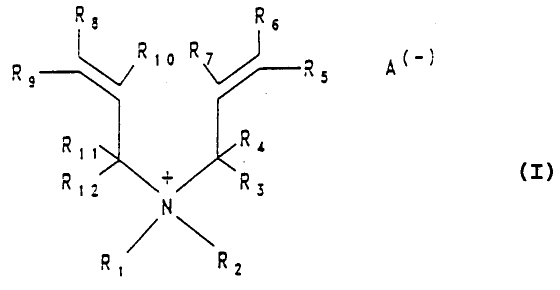 or mixture or mixed crystal thereof, in which the radicals
R₃ to R₁₂ independently of one another are in each case a hydrogen atom, a halogen atom, a hydroxyl radical, a primary, secondary or tertiary amino radical, a carboxylic acid or carboxylic acid ester radical, an acyl radical, a sulfonic acid or sulfonic acid ester radical, a cyano radical or a nitro radical, or are in each case a radical based on an aromatic hydrocarbon, which can be interrupted by hetero atoms;
R₁ and R₂ have the meanings given for R₃ to R₁₂ and are an aliphatic or aromatic hydrocarbon radical, which can be interrupted by hetero atoms;
A^{⊖} is in each case the stoichiometric equivalent of an inorganic anion from the group comprising F⁻, I⁻, OH⁻, HSO₄⁻, S₂⁻, SO₃²⁻, S₂O₃²⁻, HCO₃⁻, CO₃²⁻, H₂PO₄⁻, HPO₄²⁻, PO₄³⁻, CN⁻, cyanate, isocyanate, zinc tetracyanate, zinc tetrathiocyanate, perchlorate, PF₆⁻, MoO₄²⁻, MoS₄²⁻ and WO₄²⁻, or a combination thereof, or of an anion of a heteropolyacid or of a borate of the formula (II) 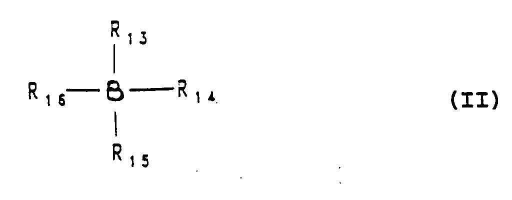 in which the radicals R₁₃ to R₁₆ on the borate anion independently of one another are aliphatic or cycloaliphatic radicals, or aryl or heteroaryl or aralkyl radicals, it being possible for these radicals to be substituted by alkyl (C₁-C₄), alkoxy(C₁-C₄) or aryl radicals or by halogen atoms, or are fluorine atoms, or of an organic anion based on a phenolate, olefinic, aliphatic or aromatic carboxylate, sulfonate, thiolate or sulfate, in which the alkyl, alkenyl or aryl radicals can also be perfluorinated, or based on a disulfo-pyrrolidinium-betaine of the formula (III) 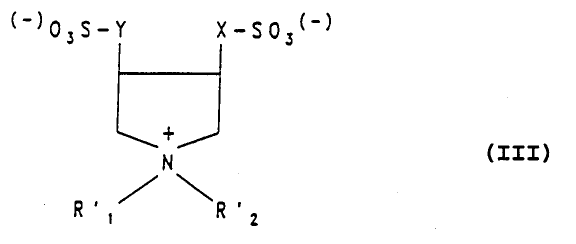 in which R₁' and R₂' have the meanings given for R₁ and R₂, and X and Y are in each case a straight-chain or branched aliphatic, saturated or unsaturated alkyl(C₁-C₁₈) radical or alkoxy(C₁-C₁₈) radical, or a mixture of such compounds and mixed crystals with mixed anions and/or cations, with the exclusion of the compounds di(2-propenyl)ammonium perchlorate, di(2-propenyl)ammonium tosylate, di(2-propenyl)ammonium fluoride and di(2-propenyl)ammonium tetrafluoroborate.

2. A compound as claimed in claim 1, wherein the anion A^{⊖} is a combination of the anions mentioned with Cl⁻, Br⁻ and/or SO₄²⁻.

3. A compound as claimed in claim 1, wherein the anion A^{⊖} is an anion of a heteropolyacid, preferably P(Mo₃O₁₀)₄³⁻, P(W₃O₁₀)₄³⁻ or a silicomolybdate.

4. A compound as claimed in claim 1, wherein the anion A^{⊖} is tetrafluoroborate, tetrachloroborate, tetraphenylborate, tetra(fluorophenyl)borate, tetra(chlorophenyl)borate, tetratolylborate, tetranaphthylborate, tetra(methoxyphenyl)borate, tetrabiphenylborate, tetrabenzylborate or tetrapyridylborate, or a combination thereof.

5. A compound as claimed in claim 1, wherein the anion A^{⊖} is an organic anion from the group comprising ethylsulfate, phenolate, nitrophenolate, thiolate, acetate, lactate, benzoate, the mono- or dianion of dithiodibenzoic acid, salicylate, 2-hydroxy-3-naphthoate, 2-hydroxy-6-naphthoate, ethylsulfonate, phenylsulfonate, tosylate, perfluoroacetate, perfluoro-C₁-C₃₀-alkylbenzoate, perfluoroethylsulfonate, perfluoro-C₁-C₃₀-alkylbenzenesulfonate and saturated or unsaturated aliphatic or cycloaliphatic or aromatic di- and tricarboxylate or di- or trisulfonate, preferably citrate, oxalate or succinate.

6. A compound as claimed in claim 1, wherein the anion A^{⊖} is a disulfo-pyrrolidinium-betaine of the formula (III), in which R₁' and R₂' are R₁ and R₂, and X and Y are straight-chain or branched aliphatic, saturated or unsaturated alkyl(C₁-C₁₈) or alkoxy(C₁-C₁₈) radicals, preferably alkyl(C₁-C₅) or alkoxy (C₁-C₅) radicals, or polyoxyalkylene radicals, preferably polyoxyethylene and polyoxypropylene radicals, of the formula (alkylene(C₂-C₅)-O)ₙ-R, in which R is a hydrogen atom or an alkyl(C₁-C₄) radical and n is a number from 1 to 10.

7. A compound as claimed in claim 1, wherein the anion A^{⊖} is an anion from the group comprising F⁻, I⁻, BF₄⁻, B(aryl)₄⁻, PF₆⁻ or P(Mo₃O₁₀)₄³⁻, or of a disulfo-pyrrolidinium-betaine of the formula (III);
R₁ and R₂, and R₁' and R₂'
independently of one another are hydrogen atoms, straight-chain or branched, saturated or unsaturated alkyl(C₁-C₁₈) or alkoxy(C₁-C₁₈) radicals, polyoxyalkylene radicals, preferably polyoxyethylene and/or polyoxypropylene radicals, of the formula (alkylene(C₁-C₅)-O)ₙ-R, in which R is a hydrogen atom, an alkyl(C₁-C₄) radical or an acyl radical, preferably the acetyl, benzoyl or naphthoyl radical, and n is a number from 1 to 10; aryl or heteroaryl radicals, preferably phenyl, naphthyl or pyridyl radicals; aralkyl radicals, preferably tolyl radicals; aralkoxy radicals, preferably methoxyphenyl radicals; alkaryl radicals, preferably benzyl radicals; or cycloalkyl radicals, preferably cyclopentyl or cyclohexyl radicals, or the abovementioned radicals can contain at least one hetero atom, preferably nitrogen, oxygen, sulfur, phosphorus or a combination thereof, and can be substituted by one or more carboxamide radicals, sulfonamide radicals, urethane radicals, keto radicals, primary, secondary or tertiary amino radicals, nitro radicals, ether radicals, in particular alkylene(C₂-C₄)-O-alkyl(C₁-C₄) radicals, alkyl(C₁-C₄) radicals, alkoxy(C₁-C₄) radicals, aroxy radicals, in particular phenoxy radicals, halogenoalkyl(C₁-C₃₀) radicals, halogenoalkoxy(C₁-C₃₀) radicals or ester radicals, in particular -C(O)O-alkyl(C₁-C₄), one or more halogen atoms or hydroxyl, carboxyl, sulfonic acid, cyano or mercapto groups, or a combination thereof,
or R₁ and R₂, and R₁' and R₂'
together form a saturated or unsaturated, aromatic or non-aromatic 5- to 7-membered ring system, preferably the pyridinium ring system, which can contain further hetero atoms, preferably nitrogen, oxygen, sulfur or a combination thereof, in the ring, in particular the morpholinium ring system, and which can be substituted and/or modified by condensation of or bridging to other ring systems, in particular the quinolinium ring system; and
R₃ to R₁₂
independently of one another are hydrogen, hydroxyl, carboxyl, sulfo, cyano, mercapto, halogen atoms or aroxy radicals, preferably phenoxy radicals.

8. A compound as claimed in claim 1 or 7, wherein A^{⊖} is an anion from the group comprising BF₄⁻ and B(aryl)₄⁻, in which aryl is phenyl, naphthyl, fluorophenyl, chlorophenyl, methoxyphenyl, biphenyl, pyridyl or tolyl or a combination thereof;
R₁ and R₂
independently of one another are hydrogen atoms, straight-chain or branched, saturated or unsaturated alkyl(C₁-C₈) or alkoxy(C₁-C₈) radicals, aryl or heteroaryl radicals, in particular phenyl, naphthyl or pyridyl radicals, aralkyl radicals, in particular tolyl radicals, aralkoxy radicals, in particular methoxyphenyl radicals, alkaryl radicals, in particular benzyl radicals, or cycloalkyl radicals, in particular cyclopentyl or cyclohexyl radicals,
it being possible for the radicals R₁ and R₂ to be substituted by one or more halogen atoms, hydroxyl, carboxyl, sulfonic acid or carboxamide radicals, in particular -NH-C(O)-alkyl(C₁-C₄), sulfonamide radicals, in particular -NH-SO₂-alkyl(C₁-C₄), keto radicals, in particular -C(O)-alkyl(C₁-C₄), primary, secondary or tertiary amino radicals, in particular -NH₂, -NH[alkyl(C₁-C₄)]- or -N[alkyl(C₁-C₄)]₂, nitro radicals, ether radicals, in particular alkylene(C₂-C₄)-O-alkyl(C₁-C₄), alkyl(C₁-C₄) radicals, alkoxy(C₁-C₄) radicals, aroxy radicals, in particular phenoxy radicals, halogenoalkyl(C₁-C₄) radicals, halogenoalkoxy(C₁-C₄) radicals or ester radicals, in particular -C(O)O-alkyl(C₁-C₄); and
R₃ to R₁₂
independently of one another are hydrogen, hydroxyl or halogen atoms.

9. A compound as claimed in at least one of claims 1, 4 and 6 to 8, wherein A^{⊖} has the meaning BF₄⁻, B(phenyl)₄⁻ or disulfo-pyrrolidinium-betaine of the formula (III), in which R₁' and R₂' have the meanings given below for R₁ and R₂, R₁ is hydrogen or methyl, R₂ is hydrogen, methyl or octyl, and R₃ to R₁₂ are in each case hydrogen.

10. A mixed crystal or mixture of two or more compounds as claimed in at least one of claims 1 to 9.

11. A process for the preparation of a compound as claimed in at least one of claims 1 to 10, which comprises adding one or more compound(s) on which the anion A^{⊖} is based, preferably the sodium salt, to a diallylammonium halide of the formula (IV) 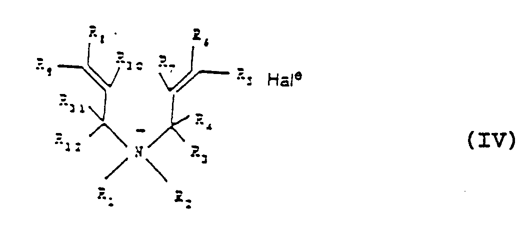 in which Hal is fluorine, chlorine, bromine or iodine as a solution in water or in a mixture of water and an organic solvent which is completely or partly miscible with water, at a temperature between 0°C and 100°C, preferably 10°C and 70°C, and a pH of between 3 and 10, preferably 5 and 8, and then, if appropriate, precipitating the compound of the formula (I) according to the invention by salting out with halogen-containing salts, for example potassium chloride.

12. The process as claimed in claim 11, wherein the reaction is carried out in a mixture of water and isopropanol, water and isobutanol or water and methyl isobutyl ketone.

13. The process as claimed in claim 11 or 12, wherein the diallylammonium chloride is reacted with sodium tetraphenylborate, sodium tetra-o-fluorophenylborate, sodium tetra-m-fluorophenylborate, sodium tetra-p-fluorophenylborate, sodium tetra-o-chlorophenylborate, sodium tetra-m-chlorophenylborate, sodium tetra-p-chlorophenylborate, sodium tetra-o-tolylborate, sodium tetra-m-tolylborate, sodium tetra-p-tolylborate, sodium tetra-1-naphthylborate, sodium tetra-2-naphthylborate, sodium tetra-o-methoxyphenylborate, sodium tetra-m-methoxyphenylborate, sodium tetra-p-methoxyphenylborate, sodium tetra-o-biphenylborate, sodium tetra-m-biphenylborate, sodium tetra-p-biphenylborate, sodium tetrabenzylborate, sodium tetra-o-pyridylborate, sodiumtetra-m-pyridylborate, sodium p-pyridylborate or sodium tetrafluoroborate.

14. The use of a compound as claimed in at least one of claims 1 to 10 as a charge control agent in electrophotographic toners and developers which are employed for copying or duplication of masters and for printing electronically, magnetically or optically stored information or in colorproofing, and as a charge control agent in powders and powder paints.

15. The use as claimed in claim 14, wherein the ionic monomeric diallylammonium compound is employed, by itself or in a combination, in a concentration of about 0.01 to about 30, preferably 0.1 to 5.0, percent by weight.

16. The use of a compound as claimed in at least one of claims 1 to 10, wherein this compound is employed as a charge-improving agent in the form of coatings of carriers or a constituent of coatings of carriers which are used in developers for electrophotographic copying or duplication of masters and for printing electronically, optically or magnetically stored information or in colorproofing.

17. The use of a compound as claimed in at least one of claims 1 to 10, wherein this compound is employed by itself or in a combination, as a charge-improving agent in powders and paints for surface coating of objects of metal, wood, plastic, glass, ceramic, concrete, textile material, paper or rubber, in particular in triboelectrically or electrokinetically sprayed powder paints.

18. The use as claimed in at least one of claims 14 to 17, wherein the anion A^{⊖} in the compound of the formula (I) is chloride, bromide or the stoichiometric equivalent of sulfate.

## Revendications

1. Composés monomères ionogènes de diallylammonium de formule générale (I) 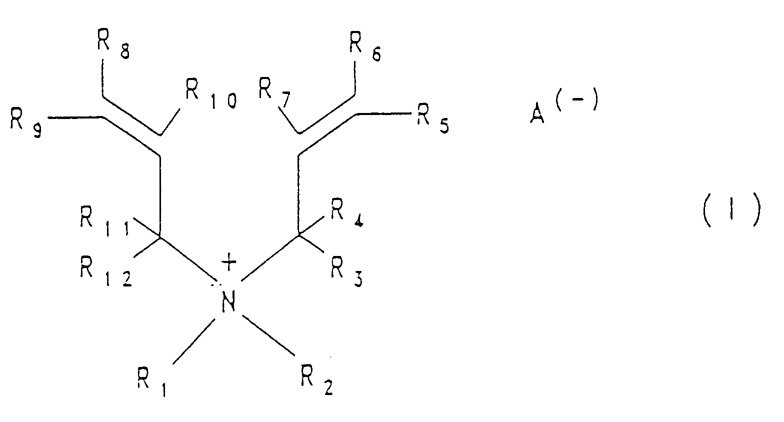 ainsi que leurs mélanges ou solutions solides,
où
les radicaux R₃ à R₁₂, indépendamment les uns des autres, représentent chacun un atome d'hydrogène, un atome d'halogène, un groupe hydroxyle, un groupe amino primaire, secondaire ou tertiaire, un résidu acide carboxylique ou ester d'acide carboxylique, un groupe acyle, un résidu acide sulfonique ou ester d'acide sulfonique, un groupe cyano ou nitro, ou encore représentent chacun un résidu à base d'un hydrocarbure aromatique, qui peut être interrompu par des hétéroatomes ;
R₁ et R₂ ont les significations données pour R₃ à R₁₂, et représentent chacun un radical hydrocarboné aliphatique ou aromatique pouvant être interrompu par des hétéroatomes;
A⁻ représente dans tous les cas l'équivalent stoechiométrique d'un anion minéral choisi parmi l'ensemble constitué des anions F⁻, I⁻, OH⁻, HSO₄⁻, S₂⁻, SO₃²⁻, S₂O₃²⁻, HCO₃⁻, CO₃²⁻, H₂PO₄⁻, HPO₄²⁻, PO₄³⁻, CN⁻, cyanate, isocyanate, zinc-tétracyanate, perchlorate, PF₆⁻, MoO₄²⁻, MoS₄²⁻, WO₄²⁻ ou une de leurs combinaisons, ou encore d'un anion, d'un hétéropolyacide ou d'un borate de formule générale (II) 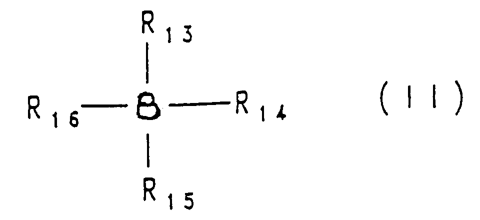 où les radicaux R₃ à R₁₆ représentent sur l'anion borate, indépendamment les uns des autres, des résidus aliphatiques ou cycloaliphatiques, des radicaux aryle ou hétéroaryle ou aralkyle, ces radicaux pouvant être substitués par des substituants alkyle en C₁-C₄, alcoxy en C₁-C₄, aryle ou alogéno, ou encore des atomes de fluor, ou d'un anion organique, à base d'un phénolate, d'un thiolate, d'un sulfonate d'un sulfate ou d'un carboxylate oléfinique aliphatique ou aromatique,dans lesquels les radicaux alkyle, alcényle ou aryle peuvent aussi être perfluorés,
ou à base d'une disulfo-pyrrolidinium-bétaïne de formule générale (III) 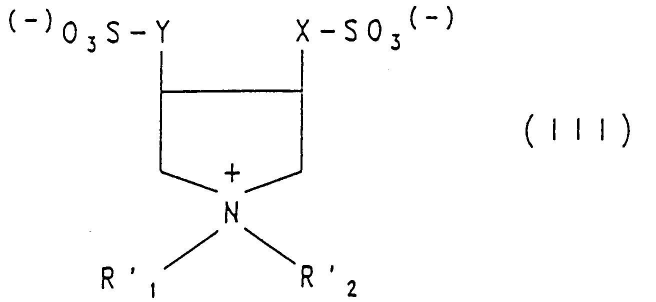 dans laquelle R₁' et R₂' ont les significations données pour R₁ et R₂, et X et Y représentent chacun un radical alkyle en C₁-C₁₈ ou alcoxy en C₁-C₁₈, aliphatique, saturé ou insaturé, à chaîne droite ou ramifiée, ainsi que les mélanges de ces composés et de solutions solides avec des anions et/ou des cations mélangés, à l'exception des composés perchlorate de di(2-propényl)ammonium, tosylate de di(2-propényl)ammonium, fluorure de di(2-propényl)ammonium et tétrafluoroborate de di(2-propényl)ammonium.

2. Composé selon la revendication 1, caractérisé en ce que l'anion A⁻ est une combinaison des anions mentionnés et de Cl⁻, Br⁻ et/ou SO₄²⁻.

3. Composé selon la revendication 1, caractérisé en ce que l'anion A⁻ est un anion d'un hétéropolyacide, de préférence, P(Mo₃O₁₀)₄³⁻, P(W₃O₁₀)₄³⁻ ou un silicomolybdate.

4. Composé selon la revendication 1, caractérisé en ce que l'anion A⁻ est l'anion tétrafluoroborate, tétrachloroborate, tétraphénylborate, tétra(fluorophényl)borate, tétra(chlorophényl)borate, tétratolylborate, tétranaphtylborate, tétra(méthoxyphényl)borate, tétrabiphénylborate, tétrabenzylborate, tétrapyridylborate ou une de leurs combinaisons.

5. Composé selon la revendication 1, caractérisé en ce que l'anion A⁻ est un anion organique choisi parmi les anions éthylsulfate, phénolate, nitrophénolate, thiolate, acétate, lactate, benzoate, le monoanion ou le dianion de l'acide dithiodibenzoïque, salicylate, 2-hydroxy-3-naphtoate, 2-hydroxy-6-naphtoate, éthylsulfonate, phénylsulfonate, tosylate, perfluoracétate, perfluoro-(alkyle en C₁-C₃₀)benzoate, perfluoréthylsulfonate, perfluoro-(alkyle en C₁-C₃₀)benzènesulfonate, di- ou tricarboxylate ou di- ou trisulfonate aliphatique, saturé ou insaturé, ou cycloaliphatique ou aromatique, de préférence citrate, oxalate ou succinate.

6. Composé selon la revendication 1, caractérisé en ce que l'anion A⁻ est une disulfo-dipyrrolidiniumbétaïne de formule (III), où R₁' et R₂' sont égaux à R₁ et R₂, et X et Y sont des radicaux alkyle en C₁-C₁₈ ou alcoxy en C₁-C₁₈ aliphatiques à chaîne droite ou ramifiée, saturée ou insaturée, de préférence des radicaux alkyle en C₁-C₅ ou alcoxy en C₁-C₅, des résidus polyoxyalkylène, de préférence polyoxyéthylène et polyoxypropylène de formule générale ((alkylène en C₂-C₅)-O)ₙ-R, où R est un atome d'hydrogène ou un groupe alkyle en C₁-C₄, et n est un nombre de 1 à 10.

7. Composé selon la revendication 1, caractérisé en ce que l'anion A⁻ est un anion choisi dans l'ensemble F⁻, I⁻, BF₄⁻, B(aryle)₄⁻, PF₆⁻, P(Mo₃O₁₀)₄³⁻ ou d'une disulfo-pyrrolidinium-bétaïne de formule (III) ;
R₁ et R₂, ou R₁' et R₂', indépendamment les uns des autres, représentent des atomes d'hydrogène, des radicaux alkyle en C₁-C₁₈ ou alcoxy en C₁-C₁₈ saturés ou insaturés, à chaîne droite ou ramifiée, des résidus polyoxyalkylène, de préférence polyoxyéthylène et/ou polyoxypropylène de formule générale ((alkylène en C₁-C₅)-O)ₙ-R, où R est un atome d'hydrogène, un groupe alkyle en C₁-C₄ ou un groupe acyle, de préférence le groupe acétyle, benzoyle ou naphtoyle, et n est un nombre de 1 à 10 ; des radicaux aryle ou hétéroaryle, de préférence phényle, naphtyle ou pyridyle ; des radicaux aralkyle, de préférence tolyle ; des radicaux aralcoxy, de préférence méthoxyphényle ; des radicaux alkaryle, de préférence benzyle ; ou des radicaux cycloalkyle, de préférence cyclopentyle ou cyclohexyle, ou encore les radicaux mentionnés ci-dessus contiennent au moins un hétéroatome, de préférence l'azote, l'oxygène, le soufre, le phosphore ou une de leurs combinaisons, ou encore peuvent être substitués par un ou plusieurs radicaux carboxamide, sulfonamide, uréthanne, céto, amino primaire, secondaire ou tertiaire, nitro, éther, en particulier (alkylène en C₂-C₄)-O-(alkyle en C₁-C₄) et alkyle en C₁-C₄, alcoxy en C₁-C₄, aroxy, en particulier phénoxy, halogénalkyle en C₁-C₃₀, halogénalcoxy en C₁-C₃₀, ester, en particulier -C(O)O-alkyle en C₁-C₄, par un ou plusieurs atomes d'halogène, groupes hydroxyle, carboxyle, acide sulfonique, cyano ou mercapto, ou une de leurs combinaisons,
ou bien R₁ et R₂, ou R₁' et R₂', forment ensemble un système cyclique à 5 à 7 chaînons, saturés ou insaturés, aromatiques ou non aromatiques, de préférence le système cyclique pyridinium, qui peut contenir dans son noyau d'autres hétéroatomes, de préférence des atomes d'azote, d'oxygène, de soufre ou une de leurs combinaisons, en particulier le système cyclique morpholinium, et qui peut être substitué et/ou modifié par condensation d'autres systèmes cycliques ou pontage à d'autres systèmes cycliques, en particulier le système cyclique quinoléinium ; et
R₃ à R₁₂, indépendamment les uns des autres, sont des hydrogènes ou des radicaux hydroxy, carboxy, sulfo, cyano, mercapto ou halogéno ou aroxy, de préférence des groupes phénoxy.

8. Composé selon la revendication 1 ou 7, caractérisé en ce que A⁻ est un anion choisi parmi les anions BF₄⁻ ou B(aryle)₄⁻, où aryle désigne les radicaux phényle, naphtyle, fluorophényle, chlorophényle, méthoxyphényle, biphényle, pyrydile ou tolyle, ou une de leurs combinaisons ;
R₁ et R₂, indépendamment l'un de l'autre, sont des atomes d'hydrogène, des radicaux alkyle en C₁-C₈ ou alcoxy en C₁-C₈ à chaîne droite ou ramifiée, saturés ou insaturés, des radicaux aryle ou hétéroaryle, en particulier phényle, naphtyle ou pyridyle, des radicaux aralkyle, en particulier tolyle, des radicaux aralcoxy, en particulier méthoxyphényl, des radicaux alkaryle, en particulier benzyle, ou des radicaux cycloalkyle, en particulier cyclopentyle ou cyclohexyle, où les radicaux R₁ et R₂ peuvent être substitués par un ou plusieurs atomes d'halogène, radicaux hydroxyle, carboxyle, acide sulfonique ou carboxamide, en particulier NH-C(O)-alkyle en C₁-C₄, sulfonamido, en particulier -NH-SO₂-alkyle en C₁-C₄, céto, en particulier -C(O)-alkyle en C₁-C₄, amino primaire, secondaire ou tertiaire, en particulier -NH₂, -NH[alkyle en C₁-C₄], -N[alkyle en C₁-C₄]₂, radicaux nitro, éther, en particulier (alkylène en C₂-C₄)-O-(alkyle en C₁-C₄), alkyle en C₁-C₄, alcoxy en C₁-C₄, aroxy, en particulier phénoxy, halogénalkyle en C₁-C₄, halogénalcoxy en C₁-C₄ ou ester, en particulier -C(O)-alkyle en C₁-C₄ ;
R₃ à R₁₂, indépendamment les uns des autres, sont des hydrogènes, des groupes hydroxy ou des atomes d'halogène.

9. Composé selon au moins l'une des revendications 1, 4 et 6 à 8, caractérisé en ce que A⁻ représente BF₄⁻, B(phényle)₄⁻ ou disulfopyrrolidinium-bétaïne de formule (III) où R₁' et R₂' ont les significations données ci-après pour R₁ et R₂, R₁ est un hydrogène ou le groupe méthyle, R₂ est un hydrogène ou le groupe méthyle ou octyle et R₃ à R₁₂ sont chacun des hydrogènes.

10. Solution solide ou mélange d'au moins deux composés selon au moins l'une des revendications 1 à 9.

11. Procédé pour préparer un composé selon au moins l'une des revendications 1 à 10, caractérisé en ce qu'on ajoute à un halogénure de diallylammonium de formule (IV) 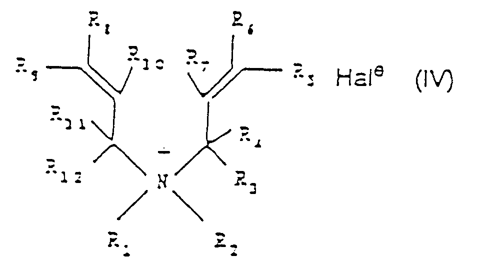 dans laquelle Hal est le fluor, le chlore, le brome ou l'iode, dissous dans l'eau ou dans un mélange d'eau et d'un solvant organique complètement ou partiellement miscible à l'eau, un ou plusieurs composés à base de l'anion A⁻, de préférence leurs sels de sodium, à une température de 0 à 100°C et de préférence de 10 à 70°C, et à un pH compris entre 3 et 10 et de préférence de 5 à 8, puis on provoque éventuellement la précipitation du composé selon l'invention de formule (I), par relargage avec des sels halogénés, par exemple le chlorure de potassium.

12. Procédé selon la revendication 11, caractérisé en ce qu'on procède à la réaction dans un mélange d'eau et d'isopropanol, d'eau et d'isobutanol ou d'eau et de méthylisobutylcétone.

13. Procédé selon la revendication 11 ou 12, caractérisé en ce que le chlorure de diallylammonium est mis à réagir avec du tétraphénylborate de sodium, du tétra-o-fluorophénylborate de sodium, du tétra-m-fluorophénylborate de sodium, du tétra-p-fluorophénylborate de sodium, du tétra-o-chlorophénylborate de sodium, du tétra-m-chlorophénylborate de sodium, du tétra-p-chlorophénylborate de sodium, du tétra-o-tolylborate de sodium, du tétra-m-tolylborate de sodium, du tétra-p-tolylborate de sodium, du tétra-1-naphtylborate de sodium, du tétra-2-naphtylborate de sodium, du tétra-o-méthoxyphénylborate de sodium, du tétra-m-méthoxyphénylborate de sodium, du tétra-p-méthoxyphénylborate de sodium, du tétra-o-biphénylborate de sodium, du tétra-m-biphénylborate de sodium, du tétra-p-biphénylborate de sodium, du tétrabenzylborate de sodium, du tétra-o-pyridylborate de sodium, du tétra-m-pyridylborate de sodium, du tétra-p-pyridylborate de sodium ou du tétrafluoroborate de sodium.

14. Utilisation d'un composé selon au moins l'une des revendications 1 à 10 comme régulateur de charge dans des toners et révélateurs électrophotographiques, qui sont utilisés pour copier ou polycopier des originaux, et pour imprimer des informations mémorisées par un procédé électronique, magnétique ou optique, ou encore dans le cadre d'épreuves couleurs, et aussi comme régulateur de charge dans des poudres et peintures en poudre.

15. Utilisation selon la revendication 14, caractérisée en ce qu'on utilise le composé de diallylammonium monomère ionogène seul ou en combinaison, à une concentration d'environ 0,01 à environ 30 et de préférence de 0,1 à 5,0 % en poids.

16. Utilisation d'un composé selon au moins l'une des revendications 1 à 10, caractérisé en ce que ces composés, sous forme d'agents améliorant la charge, sont utilisés sous forme de revêtements de porteurs ou de constituant de revêtements de porteurs, qui sont utilisés dans les révélateurs pour la copie électrophotographique, pour la copie ou la polycopie électrophotographique d'originaux, et pour imprimer des informations mémorisées par un procédé électronique, optique ou magnétique, ou dans le cadre d'une épreuve en couleurs.

17. Utilisation d'un composé selon au moins l'une des revendications 1 à 10, caractérisé en ce que ces composés sont utilisés seuls ou en combinaison, en tant qu'agents améliorant la charge dans des poudres et peintures pour revêtement superficiel d'objets en métal, en bois, en plastique, en verre, en céramique, en béton, en un matériau textile, en papier ou en caoutchouc, en particulier dans les peintures en poudre pojetées par un procédé triboélectrique ou électrocinétique.

18. Utilisation d'un composé selon au moins l'une des revendications 14 à 17, caractérisé en ce que l'anion A⁻, dans le composé de formule (I), est l'anion chlorure, bromure, ou l'équivalent stoechimétrique de l'anion sulfate.
